(19) Europäisches Patentamt
European Patent Office
Office européen des brevets

(11) EP 1 869 001 B1

(12) **EUROPÄISCHE PATENTSCHRIFT**

(45) Veröffentlichungstag und Bekanntmachung des Hinweises auf die Patenterteilung:
**12.01.2011 Patentblatt 2011/02**

(21) Anmeldenummer: **06723484.9**

(22) Anmeldetag: **17.03.2006**

(51) Int Cl.:
***C07D 217/26*** (2006.01) ***C07D 403/12*** (2006.01)
***A61K 31/472*** (2006.01) ***A61K 31/4725*** (2006.01)
***A61P 19/02*** (2006.01)

(86) Internationale Anmeldenummer:
**PCT/EP2006/002440**

(87) Internationale Veröffentlichungsnummer:
**WO 2006/102998 (05.10.2006 Gazette 2006/40)**

# (54) SUBSTITUIERTE TETRAHYDROISOCHINOLINE ALS MMP-INHIBITOREN, VERFAHREN ZU IHRER HERSTELLUNG UND IHRE VERWENDUNG ALS MEDIKAMENT

SUBSTITUTED TETRAHYDROISOCHINOLINES AS MMP INHIBITORS, RELATED PRODUCTION METHOD AND USE AS MEDICINE

TETRAHYDROISOQUINOLEINES SUBSTITUEES EN TANT QU'INHIBITEURS DE MMP, PROCEDE DE PRODUCTION ASSOCIE ET UTILISATION COMME MEDICAMENT

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **31.03.2005 DE 102005015040**

(43) Veröffentlichungstag der Anmeldung:
**26.12.2007 Patentblatt 2007/52**

(73) Patentinhaber: **Sanofi-Aventis**
**75013 Paris (FR)**

(72) Erfinder:
- **HOFMEISTER, Armin**
**65926 Frankfurt am Main (DE)**
- **SCHUDOK, Manfred**
**65926 Frankfurt am Main (DE)**
- **MATTER, Hans**
**65926 Frankfurt am Main (DE)**
- **BREITSCHOPF, Kristin**
**65926 Frankfurt am Main (DE)**
- **UGOLINI, Antonio**
**F-94403 Vitry-sur-Seine (FR)**

(74) Vertreter: **Löschner, Thomas et al**
**Sanofi-Aventis Deutschland GmbH**
**Patent- und Lizenzabteilung**
**Industriepark Höchst**
**Gebäude K 801, Raum 5041**
**D-65926 Frankfurt am Main (DE)**

(56) Entgegenhaltungen:
**WO-A-97/18194** **WO-A-03/016248**
**DE-A-102004 031 850** **US-A- 5 962 471**

- **DAWEI MA ET. AL.: "Tetrahydroisoquinoline based sulfonamide hydroxamates as potent matrix metalloproteinase inhibitors." BIOORGANIC AND MEDICINAL CHEMISTRY LETTERS, Bd. 14, Nr. 1, 2004, Seiten 47-50, XP002385132 in der Anmeldung erwähnt**

EP 1 869 001 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

In Erkrankungen wie Osteoarthritis und Rheuma findet eine Zerstörung des Gelenkes statt, besonders bedingt durch den proteolytischen Abbau von Kollagen durch Kollagenasen. Kollagenasen gehören zur Superfamilie der Metalloproteinasen (MP) bzw. Matrix-Metallproteinasen (MMP bzw. MMP's). Die MMP's bilden eine Gruppe von Zn-abhängigen Enzymen, die am biologischen Abbau der extrazellulären Matrix beteiligt sind (D. Yip et al., Investigational New Drugs 1999, 17, 387-399 und Michaelides et al., Current Pharmaceutical Design 1999, 5, 787-819). Diese MMP's sind insbesondere fähig, fibrilläres und nicht-fibrilläres Kollagen, sowie Proteoglycane abzubauen, die beide wichtige Matrixbestandteile darstellen. MMP's sind beteiligt an Prozessen der Wundheilung, der Tumorinvasion, Metastasenwanderung sowie an Angiogenese, multipler Sklerose und Herzversagen (Michaelides et al., ibid, Seite 788). Insbesondere spielen sie eine wichtige Rolle beim Abbau der Gelenkmatrix in der Arthrose und der Arthritis, sei es nun die Osteoarthrose, Osteoarthritis oder die rheumatoide Arthritis.

Die Aktivität der MMP's ist weiterhin essentiell für viele der Prozesse, die bei der atherosklerotischen Plaque-Bildung eine Rolle spielen, wie die Infiltration inflammatorischer Zellen, der glatten Muskelzell-Migration sowie Proliferation und Angiogenese (S. J. George, Exp. Opin. Invest. Drugs 2000, 9 (5), 993-1007). Weiterhin kann die Matrix-Degradation durch MMP Plaque-Instabilitäten bis hin zu Rupturen verursachen, was zu den klinischen Symptomen der Atherosklerose, instabilen Angina Pectoris, Myokardinfarkt oder Schlaganfall führen kann (E. J. M. Creemers et al, Circulation Res. 2001, 89, 201-210). Insgesamt betrachtet kann die gesamte MMP-Familie alle Komponenten der extrazellulären Matrix der Blutgefäße abbauen; deshalb ist deren Aktivität in starkem Maße Regulationsmechanismen in normalen Blutgefäßen unterworfen. Die erhöhte MMP-Aktivität während der Plaque-Bildung und Plaque-Instabilität wird durch erhöhte Cytokin- und Growth-Faktor-stimulierte Gen-Transkription, erhöhte Zymogen-Aktivierung und eine Imbalance des MMP-TIMP-Verhältnisses verursacht (Tissue inhibitors of metalloproteases). Daher ist eine MMP-Inhibierung oder die Wiedererlangung der MMP-TIMP-Balance hilfreich in der Behandlung der atherosklerotischen Erkrankungen. Darüber hinaus werden neben der Atherosklerose auch weitere cardiovaskuläre Erkrankungen durch eine erhöhte MMP-Aktivität zumindest mitverursacht, wie beispielsweise Restenose, dilatierte Cardiomyopathie und der schon erwähnte Myokardinfarkt. Es konnte gezeigt werden, dass durch die Applikation synthetischer Inhibitoren von MMP's in experimentellen Tiermodellen dieser Erkrankungen deutliche Verbesserungen erzielt werden, z. B. betreffend die Bildung atherosklerotischer Läsionen, Neointima-Bildung, Linksventrikuläres Remodelling, Dysfunktion der Pumpleistung oder Infarkt-Heilung. In verschiedenen präklinischen Studien mit MMP-Inhibitoren zeigte sich bei detaillierter Gewebsanalyse eine reduzierte Kollagen-Schädigung, verbessertes extrazelluläres Matrix-Remodelling und eine verbesserte Struktur und Funktion von Herzmuskel und Gefäßen. Von diesen Prozessen werden insbesondere die Matrix-Remodelling-Prozesse und MMP-regulierte Fibrosen als wichtige Komponenten im Fortschreiten von Herzerkrankungen (Infarkt) angesehen (Drugs 2001, 61, 1239-1252).

MMP's spalten Matrixproteine wie Kollagen, Laminin, Proteoglykane, Elastin oder Gelatin, des weiteren prozessieren (d. h. aktivieren oder desaktivieren) MMP's durch Spaltung eine Vielzahl weiterer Proteine und Enzyme unter physiologischen Bedingungen, so dass Ihnen eine wichtige Rolle im gesamten Organismus zukommt, mit besonderer Bedeutung im Bindegewebe und Knochen.

Substituierte Tetrahydroisochinoline waren als Inhibitoren der MMP's beispielsweise in den Patentanmeldungen WO9718194, CN1380288A, WO03016248 und DE102004031850.6 sowie in Bioorg. Med. Chem. Lett. 2004, 14, 47-50 und Current Medicinal Chemistry 2001, 8, 425-474 beschrieben.

Die Verbindungen der vorliegenden Erfindung zeichnen sich in der Regel durch eine verbesserte Löslichkeit, sowie eine geringere Tendenz zur Bindung an Plasmaproteine und eine stärkere Aktivität aus, insbesondere in Serum-basierten Testsystemen. Die verbesserten Eigenschaften wurden erfindungsgemäß erreicht durch den Einbau von mindestens einer löslichkeitssteigernden Gruppe X-Q-Y, die die Lipophilie der erfindungsgemäßen Verbindungen senkt.

Die vorliegende Erfindung betrifft daher eine Verbindung der Formel (I)

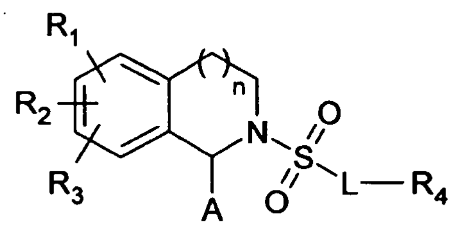

(I)

wobei

$R_1$, $R_2$ und $R_3$ unabhängig voneinander H, F, Cl, Br, CN, OH, $(C_1-C_6)$Alkyl, $(C_3-C_8)$Cycloalkyl, $O(C_1-C_6)$Alkyl, $O(C_3-C_8)$Cycloalkyl, $OC(O)-(C_1-C_6)$Alkyl, $OC(O)-(C_3-C_8)$Cycloalkyl, $C(O)O-(C_1-C_6)$Alkyl, $C(O)O-(C_3-C_8)$Cycloalkyl, $C(O)NR_5R_6$, $NR_5R_6$, $NR_5C(O)R_6$ oder eine Gruppe X-Q-Y sind, wobei
X eine kovalente Bindung, O oder $NR_7$,
Q $(C_1-C_4)$Alkylen,
Y $OR_8$, $NR_8R_9$, $C(O)OR_8$, $S(O)_2OR_8$, $S(O)_2NR_8R_9$, ein fünf- oder sechsgliedriger gesättigter Heterozyklus mit 1 oder 2 N- und/oder O-Atomen ist, in dem die N-Atome mit H oder $(C_1-C_6)$-Alkyl substituiert sind, oder Y ein fünf- oder sechsgliedriger aromatischer Heterozyklus mit 1, 2 oder 3 N-Atomen ist,

wobei 1 oder 2 Reste $R_1$, $R_2$ oder $R_3$ durch X-Q-Y beschrieben werden,

A $C(O)OR_{10}$, $C(O)NR_{10}R_{11}$ oder $C(O)NR_{10}OH$ ist,

n 1,

L definiert wird durch eine kovalente Bindung oder $(C_1-C_4)$Alkylen,

$R_4$ Phenyl oder $(C_5-C_{14})$Heteroaryl ist, wobei der Phenyl- oder $(C_5-C_{14})$Heteroarylrest substituiert ist mit einer Gruppe T-Z, wobei
T definiert ist durch eine kovalente Bindung, O, NH oder $N(C_1-C_4)$Alkyl,
Z ausgewählt ist aus der Gruppe Phenyl, $(C_5-C_{14})$Heteroaryl oder $(C_3-C_8)$Heterocycloalkyl, wobei Phenyl, $(C_5-C_{14})$Heteroaryl oder $(C_3-C_8)$Heterocycloalkyl unsubstituiert ist oder substituiert ist mit 1, 2 oder 3 Substituenten unabhängig voneinander ausgewählt aus der Gruppe F, Cl, Br, CN, OH, $(C_1-C_6)$Alkyl, $SO_2(C_1-C_6)$Alkyl, $O-(C_1-C_4)$Alkylen-$O-(C_1-C_6)$Alkyl, $O-(C_1-C_6)$Alkyl, $(C_3-C_8)$Cycloalkyl, $O(C_3-C_8)$Cycloalkyl, $(C_2-C_6)$Alkinyl, $O(C_2-C_6)$Alkinyl, oder $NR_{14}R_{15}$, wobei
$R_{14}$ und $R_{15}$ unabhängig voneinander definiert werden durch H, $(C_1-C_6)$Alkyl, $(C_3-C_8)$Cycloalkyl, $(C_2-C_6)$Alkinyl, $C(O)-(C_1-C_6)$Alkyl, $C(O)-O-(C_1-C_6)$Alkyl, $C(O)-NH-(C_1-C_6)$Alkyl, $C(O)-(C_3-C_8)$Cycloalkyl, $C(O)-O-(C_3-C_8)$Cycloalkyl, $C(O)-NH-(C_3-C_8)$Cycloalkyl, $C(O)-(C_2-C_6)$Alkinyl, $C(O)-O-(C_2-C_6)$Alkinyl oder $C(O)-NH-(C_2-C_6)$Alkinyl sind,

$R_5$, $R_6$, $R_7$, $R_8$, $R_9$, $R_{10}$, und $R_{11}$ unabhängig voneinander H oder $(C_1-C_6)$Alkyl

bedeuten,
oder deren pharmakologisch verträgliche Salze.

Ein weiterer Aspekt der Erfindung ist eine Verbindung der Formel (I), in der

$R_1$, $R_2$ und $R_3$ unabhängig voneinander H, F, Cl, Br, CN, OH, $(C_1-C_6)$Alkyl, Cyclopropyl, $O(C_1-C_6)$Alkyl, Acetyl, Propionyl, $C(O)O-(C_1-C_4)$Alkyl, $NR_5R_6$ oder eine Gruppe X-Q-Y sind, wobei
X O,
Q $(C_1-C_4)$Alkylen, und
Y $O-(C_1-C_4)$Alkyl, $NR_8R_9$, COOH, $C(O)O(C_1-C_4)$Alkyl, $SO_3H$, $S(O)_2O(C_1-C_4)$Alkyl, $SO_2NR_8R_9$, ein fünf- oder sechsgliedriger gesättigter Heterozyklus mit 1 oder 2 N- und/oder O-Atomen ist, in dem die N-Atome mit H oder $(C_1-C_4)$-Alkyl substituiert sind, oder Y ein fünf- oder sechsgliedriger aromatischer Heterozyklus mit 1 oder 2 N-Atomen ist,

wobei ein oder zwei Reste $R_1$, $R_2$ oder $R_3$ durch eine Gruppe X-Q-Y beschrieben werden,

A COOH, $C(O)NH_2$ oder C(O)NHOH ist,

n 1,

L eine kovalente Bindung ist,

$R_4$ Phenyl oder $(C_5-C_{10})$Heteroaryl ist, wobei der Phenyl- oder $(C_5-C_{10})$Heteroarylrest substituiert ist mit einer Gruppe T-Z, wobei

T definiert ist durch eine kovalente Bindung oder O,
Z ausgewählt ist aus der Gruppe Phenyl oder ($C_5$-$C_{10}$)Heteroaryl,wobei Phenyl oder ($C_5$-$C_{14}$)Heteroaryl unsubstituiert ist oder substituiert ist mit 1 oder 2 Substituenten unabhängig voneinander ausgewählt aus der Gruppe F, Cl, Br, CN, OH, ($C_1$-$C_6$)Alkyl, O-($C_1$-$C_4$)Alkylen- O-($C_1$-$C_6$)Alkyl, O-($C_1$-$C_6$)Alkyl, ($C_2$-$C_6$) Alkinyl, O($C_2$-$C_6$)Alkinyl,

$R_5$ und $R_6$ unabhängig voneinander H oder ($C_1$-$C_6$)Alkyl bedeuten,

$R_8$ und $R_9$ unabhängig voneinander H oder ($C_1$-$C_4$)Alkyl bedeuten,

oder deren pharmakologisch verträgliche Salze.

Ein weiterer bevorzugter Aspekt der Erfindung ist eine Verbindung der Formel (I), in der
$R_1$, $R_2$ und $R_3$ unabhängig voneinander H, F, Cl, Br, CN, OH, ($C_1$-$C_4$)Alkyl, O($C_1$-$C_6$)Alkyl, oder eine Gruppe X-Q-Y sind, wobei

X O,

Q ($C_1$-$C_4$)Alkylen, und

Y O-($C_1$-$C_4$)Alkyl, $NR_8R_9$, COOH, C(O)O($C_1$-$C_4$)Alkyl, $SO_3H$, $S(O)_2O$($C_1$-$C_4$)Alkyl oder ein Heterozyklus ausgewählt aus der Reihe Piperidinyl, Morpholinyl, Pyrrolidinyl, Imidazolyl, Pyrazolyl, Piperazinyl oder N-Methyl-piperazinyl ist,

wobei ein oder zwei Reste $R_1$, $R_2$ oder $R_3$ durch eine Gruppe X-Q-Y beschrieben werden,

A COOH, $C(O)NH_2$ oder C(O)NHOH ist,

n 1,

L eine kovalente Bindung ist,

$R_4$ Phenyl ist, wobei der Phenylrest substituiert ist mit einer Gruppe T-Z, wobei
T definiert ist durch eine kovalente Bindung oder O,
Z ausgewählt ist aus der Gruppe Phenyl, wobei Phenyl unsubstituiert ist oder substituiert ist mit 1 oder 2 Substituenten unabhängig voneinander ausgewählt aus der Gruppe F, Cl, Br, CN, OH, ($C_1$-$C_6$)Alkyl, O-($C_1$-$C_4$)Alkylen-O-($C_1$-$C_6$)Alkyl, O-($C_1$-$C_6$)Alkyl, ($C_2$-$C_6$)Alkinyl, O($C_2$-$C_6$)Alkinyl,

$R_8$ und $R_9$ unabhängig voneinander H oder ($C_1$-$C_4$)Alkyl bedeuten,

oder deren pharmakologisch verträgliche Salze.

Ein weiterer bevorzugter Aspekt der Erfindung sind Verbindungen der Formel (I), in denen
$R_1$, $R_2$ und $R_3$ unabhängig voneinander H, F, Cl, Br, CN, ($C_1$-$C_4$)Alkyl oder eine Gruppe X-Q-Y sind, wobei

X O,

Q ($C_2$-$C_3$)Alkylen, und

Y O-($C_1$-$C_4$)Alkyl, $NR_8R_9$ oder ein Heterozyklus ausgewählt aus der Reihe Piperidinyl, Morpholinyl, Pyrrolidinyl, Imidazolyl, Pyrazolyl, Piperazinyl oder N-Methyl-piperazinyl ist,

wobei ein Rest $R_1$, $R_2$ oder $R_3$ durch eine Gruppe X-Q-Y beschrieben wird,

A COOH oder C(O)NHOH ist,

n 1,

L eine kovalente Bindung ist,

$R_4$ Phenyl ist, wobei der Phenylrest substituiert ist mit einer Gruppe T-Z, wobei

T O ist, und

Z eine Phenylgruppe ist, wobei die Phenylgruppe unsubstituiert ist oder substituiert ist mit 1 oder 2 Substituenten unabhängig voneinander ausgewählt aus der Gruppe H, F, Cl, CN, OH, $(C_1-C_4)$Alkyl, $O(C_1-C_6)$ Alkyl, vorzugsweise F oder $O(C_1-C_6)$Alkyl,

$R_8$ und $R_9$ unabhängig voneinander H oder $(C_1-C_4)$Alkyl bedeuten,

oder deren pharmakologisch verträgliche Salze.

Sofern die Verbindungen der Formeln (I) ein oder mehrere Asymmetriezentren enthalten, so können diese unabhängig voneinander sowohl S als auch R konfiguriert sein. Die Verbindungen können als reine optische Isomere, als Diastereomere, als Racemate oder als Gemische in allen Verhältnissen derselben vorliegen.

Unter dem Begriff $(C_1-C_6)$Alkyl werden Kohlenwasserstoffreste verstanden, deren Kohlenstoffkette geradkettig oder verzweigt ist und 1 bis 6 Kohlenstoffatome enthält, beispielsweise Methyl, Ethyl, n-Propyl, Iso-Propyl, n-Butyl, Iso-Butyl, tertiär-Butyl, Pentyl, Iso-Pentyl, Neopentyl, Hexyl, 2,3-Dimethylbutyl oder Neohexyl.

$(C_1-C_6)$Alkylen-Reste sind entsprechend beispielsweise Methylen, Ethylen, n-Propylen, Iso-Propylen, n-Butylen, Iso-Butylen, tertiär-Butylen, Pentylen, Iso-Pentylen, Neopentylen, Hexylen, 2,3-Dimethylbutylen oder Neohexylen. Unter dem Begriff $(C_1-C_4)$Alkyl werden Kohlenwasserstoffreste verstanden, deren Kohlenstoffkette geradkettig oder verzweigt ist und 1 bis 4 Kohlenstoffatome enthält, beispielsweise Methyl, Ethyl, n-Propyl, Iso-Propyl, Iso-Butyl, Butyl oder tertiär-Butyl.

$(C_1-C_4)$Alkylen-Reste sind entsprechend beispielsweise Methylen, Ethylen, n-Propylen, Iso-Propylen, n-Butylen, Iso-Butylen, tertiär-Butylen. Eine $(C_1-C_6)$Alkyl-oder Alkylengruppe, in der ein oder mehrere H-Atome durch F-Atome ersetzt sind, ist beispielsweise Trifluorethyl, Trifluorethyl, Trifluormethylen oder Trifluorethylen. Eine $O(C_1-C_6)$Alkylgruppe beispielsweise Methoxy, Ethoxy. Eine $O(C_1-C_6)$Alkylgruppe, in der ein oder mehrere H-Atome durch F-Atome ersetzt sind, ist beispielsweise Trifluormethoxy oder Trifluorethoxy.

Unter dem Begriff $(C_2-C_6)$Alkenyl werden Kohlenwasserstoffreste verstanden, deren Kohlenstoffkette 2 bis 6 Kohlenstoffatome enthält und je nach Kettenlänge geradkettig oder verzweigt ist und 1, 2 oder 3 Doppelbindungen aufweist, beispielsweise Ethenyl, Propenyl, Iso-Propenyl, Iso-Butenyl oder Butenyl.

$(C_2-C_6)$Alkenylen-Reste sind entsprechend beispielsweise Ethenylen, Propenylen, Iso-Propenylen, Iso-Butenylen oder Butenylen. Die Substituenten an der Doppelbindung können, sofern die prinzipielle Möglichkeit besteht, E- oder Z-ständig angeordnet sein. Die Doppelbindungen können sowohl intern als auch endständig sein.

Unter dem Begriff $(C_2-C_6)$Alkinyl werden Kohlenwasserstoffreste verstanden, deren Kohlenstoffkette 2 bis 6 Kohlenstoffatome enthält und je nach Kettenlänge geradkettig oder verzweigt ist und 1-3, vorzugsweise 1 oder 2 Dreifachbindungen aufweisen, beispielsweise Ethinyl, n-Propinyl, Iso-Propinyl, Iso-Butylinyl, Butinyl, Pentinyl oder Isomere von Pentinyl oder Hexinyl oder Isomere von Hexinyl. -$(C_2-C_6)$Alkinyl-Reste sind entsprechend beispielsweise Ethinylen, Propenylen, Iso-Propinylen, Iso-Butylinylen, Butinylen, Pentinylen. Die Dreifachbindungen können sowohl intern als auch endständig sein.

Unter dem Begriff $(C_3-C_8)$Cycloalkyl werden Reste verstanden, die sich von 3-, 4-, 5-, 6-, 7- oder 8-gliedrigen Monocyclen wie Cyclopropyl, Cyclobutyl, Cyclopentyl, Cyclohexyl, Cycloheptyl oder Cyclooctyl herleiten. Eine $(C_1-C_4)$ Alkylen-$(C_3-C_8)$Cycloalkyl-Gruppe ist eine endständige $(C_3-C_8)$CycloalkylGruppe, die über einen $(C_1-C_4)$Alkylen-Rest verbrückt ist, beispielsweise Cyclopropylmethyl.

Unter dem Begriff $(C_3-C_8)$Heterocycloalkyl werden Reste verstanden, die sich von 3-, 4-, 5-, 6-, 7- oder 8-gliedrigen Monocyclen wie Cyclopropyl, Cyclobutyl, Cyclopentyl, Cyclohexyl, Cycloheptyl oder Cyclooctyl herleiten, in denen ein oder mehrere Ringatome Sauerstoffatome, Schwefelatome oder Stickstoffatome sind, beispielsweise 1, 2, 3 oder 4 Stickstoffatome, 1 oder 2 Sauerstoffatome, 1 oder 2 Schwefelatome oder eine Kombinationen aus verschiedenen Heteroatomen, beispielsweise -einem Sauerstoffatom und einem Stickstoffatom. Die $(C_3-C_8)$Heterocycloalkyl-Reste können über alle Positionen angebunden sein, zum Beispiel über die 1-Position, 2-Position, 3-Position, 4-Position, 5-Position, 6-Position, 7-Position oder die 8-Position. Die Anbindung kann über ein C-Atom oder ein N-Atom erfolgen. $(C_3-C_8)$Heterocycloalkyl-Reste sind beispielsweise Pyrrolidinyl, Tetrahydrothiophenyl, Tetrahydrofuranyl, Piperidinyl, Pyranyl, Dioxanyl, Morpholinyl. Bevorzugt sind $(C_5-C_6)$Heterocycloalkyl-Reste, insbesondere $(C_5-C_6)$Heterocycloalkyl-Reste mit 1, 2, 3 oder 4 N- und/oder O-Atomen, in denen N-Atome durch H oder $(C_1-C_6)$Alkyl substituiert sind. Besonders bevorzugt ist Morpholinyl.

$(C_5-C_{14})$-Heteroarylreste sind aromatische mono-, bi- oder tricyclische $(C_5-C_{14})$-Ringverbindungen, in denen ein oder mehrere Ringatome Sauerstoffatome, Schwefelatome oder Stickstoffatome sind, z. B. 1, 2, 3 oder 4 Stickstoffatome, 1 oder 2 Sauerstoffatome, 1 oder 2 Schwefelatome oder eine Kombinationen aus verschiedenen Heteroatomen. $(C_5-C_{10})$-Heteroarylreste sind aromatische mono-, bi-oder tricyclische $(C_5-C_{10})$-Ringverbindungen, in denen ein oder mehrere Ringatome Sauerstoffatome, Schwefelatome oder Stickstoffatome sind, z. B. 1, 2, 3 oder 4 Stickstoffatome, 1 oder 2 Sauerstoffatome, 1 oder 2 Schwefelatome oder eine Kombinationen aus verschiedenen Heteroatomen. Die Heteroarylreste können über alle Positionen angebunden sein, zum Beispiel über 1-Position, 2-Position, 3-Position, 4-Position, 5-Position, 6-Position, 7-Position oder 8-Position. Als $(C_5-C_{10})$- bzw. $(C_5-C_{14})$Heteroarylreste gelten beispielsweise 2- oder 3-Thienyl, 2- oder 3-Furyl, 1-, 2-oder 3-Pyrrolyl, 1-, 2-, 4- oder 5-Imidazolyl, 1-, 3-, 4- oder 5-Pyrazolyl, 1,2,3-Triazol-1-, -4- oder 5-yl, 1,2,4-Triazol-1-, -3- oder -5-yl, 1- oder 5-Tetrazolyl, 2-, 4- oder 5-Oxazolyl, 3-, 4- oder 5-Isoxazolyl, 1,2,3-Oxadiazol-4- oder 5-yl, 1,2,4-Oxadiazol-3-oder 5-yl, 1,3,4-Oxadiazol-2-yl oder -5-yl, 2-, 4- oder 5-Thiazolyl, 3-, 4- oder 5-Isothiazolyl, 1,3,4-Thiadiazol-2- oder -5-yl, 1,2,4-Thiadiazol-3- oder -5-yl, 1,2,3-Thiadiazol-4- oder 5-yl, 2-, 3- oder 4-Pyridyl, 2-, 4-, 5- oder 6-Pyrimidinyl, 3- oder 4-Pyridazinyl, Pyrazinyl, 1-, 2-, 3-, 4-, 5-, 6- oder 7-Indolyl, 1-, 2-, 4- oder 5-Benzimidazolyl, 1-, 3-, 4-, 5-, 6- oder 7-Indazolyl, 2-, 3-, 4-, 5-, 6-, 7- oder 8-Chinolyl, 1-, 3-, 4-, 5-, 6-, 7- oder 8-Isochinolyl, 2-, 4-, 5-, 6-, 7- oder 8-Chinazolinyl, 3-, 4-, 5-, 6-, 7- oder 8-Cinnolinyl, 2-, 3-, 5-, 6-, 7-oder 8-Chinoxalinyl, 1-, 4-, 5-, 6-, 7- oder 8-Phthalazinyl. Umfasst sind weiterhin die entsprechenden N-Oxide dieser Verbindungen, also zum Beispiel 1-Oxy-2-, 3- oder 4-pyridyl.

Besonders bevorzugte Heteroarylreste sind die 5- oder 6-gliedrigen Heteroarylreste mit 1, 2, 3 oder 4 N-Atomen, zum Beispiel Imidazolyl, Pyrazolyl, Pyrrolyl, Triazolyl, Tetrazolyl, Thiazolyl und Oxazolyl, sowie Pyridyl und Pyrimidinyl. Desweiteren sind bevorzugt die annelierten Ringsysteme Benzofuranyl, Benzimidazolyl und Indolyl. Speziell bevorzugt ist Pyrazolyl, Indolyl und Pyridyl.

Unter pharmakologisch verträglichen Salzen von Verbindungen der Formel (I) versteht man sowohl deren organische als auch anorganische Satze, wie sie in Remington's Pharmaceutical Sciences (17. Auflage, Seite 1418 (1985)) beschrieben sind. Aufgrund der physikalischen und chemischen Stabilität und der Löslichkeit sind für saure Gruppen unter anderem Natrium-, Kalium-, Calcium- und Ammoniumsalze bevorzugt; für basische Gruppen sind unter anderem Salze der Maleinsäure, Fumarsäure, Bernsteinsäure, Äpfelsäure, Weinsäure, Methylsulfonsäure, Salzsäure, Schwefelsäure, Phosphorsäure oder von Carbonsäuren oder Sulfonsäuren geeignet, beispielsweise als Hydrochloride, Hydrobromide, Phosphate, Sulfate, Methansulfonate, Acetate, Lactate, Maleinate, Fumarate, Malate, Gluconate, sowie Salze der Aminosäuren, natürlicher Basen oder Carbonsäurenbevorzugt. Die Herstellung physiologisch verträglicher Salze aus zur Salzbildung befähigten Verbindungen der Formel (I), einschließlich deren stereoisomeren Formen, erfolgt in an sich bekannter Weise. Die Verbindungen der Formel (I) bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin, Diethanolamin oder Triethanolamin, Trometamol oder auch basischen Aminosäuren, etwa Lysin, Ornithin oder Arginin, stabile Alkali-, Erdalkali- oder gegebenenfalls substituierte Ammoniumsalze. Sofern die Verbindungen der Formel (I) basische Gruppen aufweisen, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Hemischwefel-, Phosphor-, Methansulfon-, Benzolsulfon-, p-Toluolsulfon-, 4-Brombenzol-sulfon-, Cyclohexylamidosulfon-, Trifluormethylsulfon-, 2-Hydroxyethansulfon-, Essig-, Oxal-, Wein-, Bernstein-, Glycerolphosphor-, Milch-, Äpfel-, Adipin-, Citronen-, Fumar-, Malein-, Glucon-, Glucuron- Palmitin-, oder Trifluoressigsäure in Frage.

Gegenstand der Erfindung ist ferner ein Verfahren zur Herstellung von Verbindungen der Formel (I), das wie folgt beschrieben wird.

Seitenketten vom Typ X-Q-Y werden vorzugsweise in den Positionen 5 oder 6 des Tetrahydroisochinolingerüstseingeführt. Das erfindungsgemäße Verfahren wird im folgenden an 5- bzw. 6-substituierten Verbindungen der Formel (I) beispielhaft erläutert, in denen X gleich Sauerstoff und A gleich COOH oder C(O)NHOH ist:

Im Falle der 6-substituierten Verbindungen der Formel (VIII) *vide infra* geht man von käuflichem 6-Hydroxytetrahydrolsochinolin-carbonsäure-ethylester (II) aus und setzt zunächst mit einem Sulfonsäurechlorid (III) um und erhält so die entsprechenden Sulfonamide (IV).

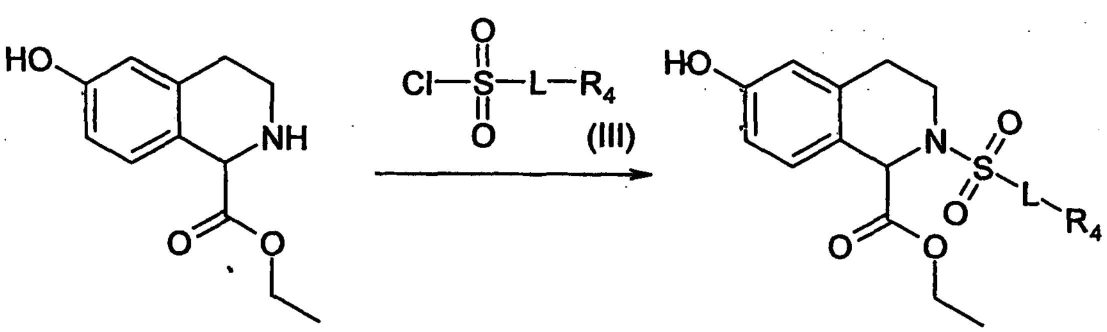

(III)

(II) (IV)

Der Aufbau der Seitenkette in der 6-Position gelingt zum Beispiel durch Umsetzung der Sulfonamide (IV) mit

zum Beispiel Diethylazodicarboxylat und Triphenylphosphin und den entsprechenden Alkoholen (V) zu den gewünschten Ethern (VI) mit hydrophiler Seitenkette in der 6-Position. Statt Diethylazodicarboxylat können andere Dialkylazodicarboxylate eingesetzt werden. Anstelle von herkömmlichem Triphenylphosphin eignen sich ebenfalls polymergebundene Analoga.

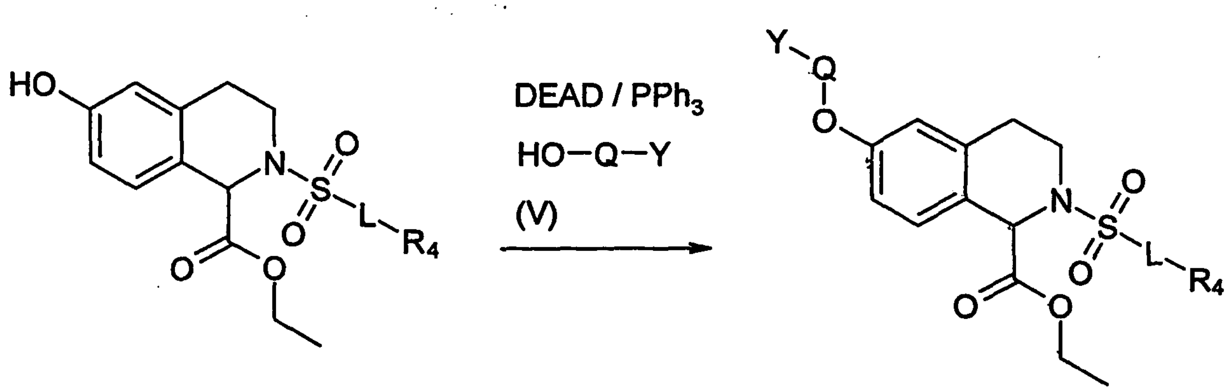


(IV) (VI)

Anschließend Verseifung der Esterfunktion liefert die Carbohsäuren (VII).

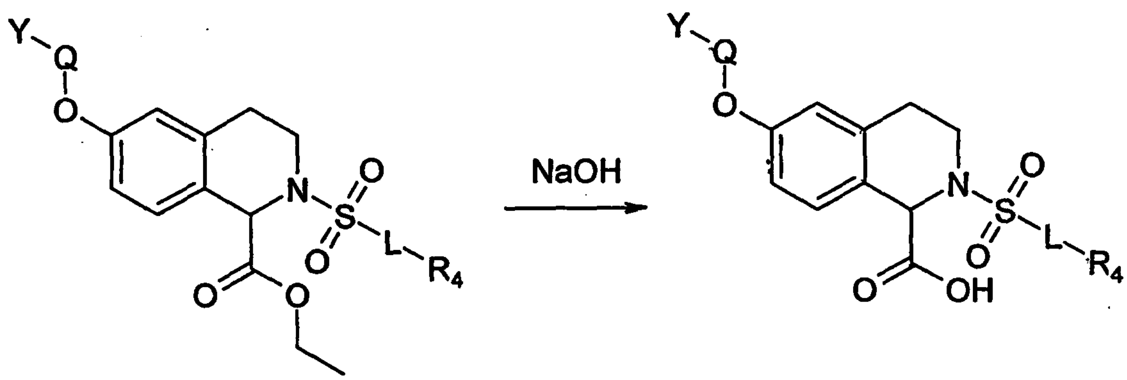


(VI) (VII)

Diese können durch dem Fachmann bekannte Methoden in die analogen Hydroxamsäuren (VIII) überführt werden.

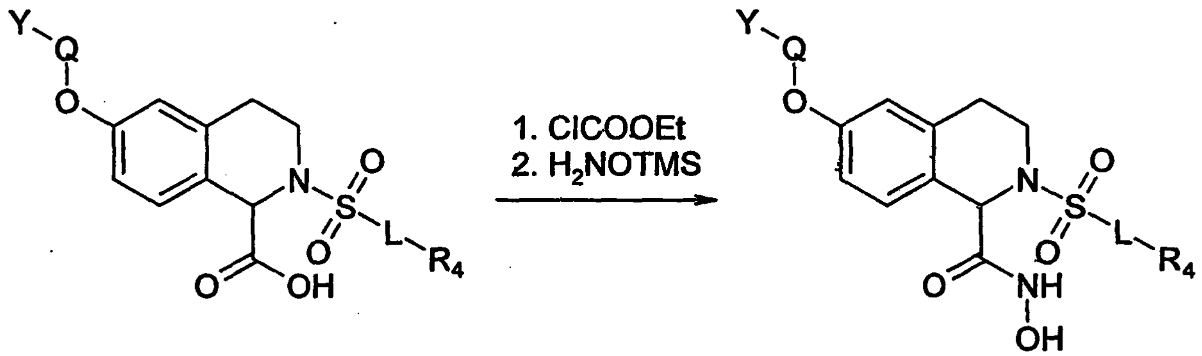


(VII) (VIII)

Im Falle der analogen 5-substituierten Verbindungen der Formel (XVI) geht man von kommerziell erhältlichem ortho-Methoxy-Phenylethylamin (IX) aus, das in der ersten Stufe zunächst mit einem Sulfonsäurechlorid (III) zur Einführung des gewünschten Rest $R_4$ umgesetzt wird, wobei man zu den Sulfonamiden (X) gelangt.

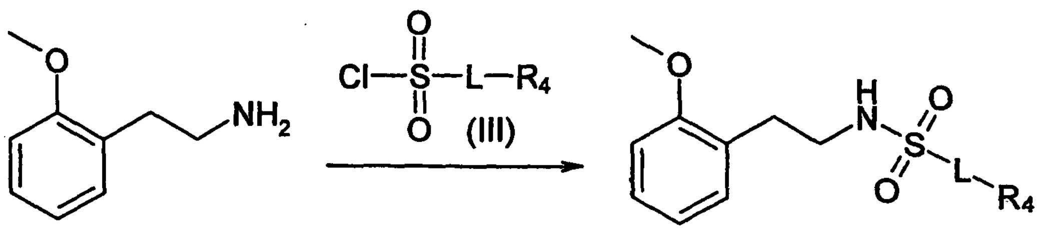


(IX) (X)

Diese können dann durch eine Pictet-Spengler Cyclisierung mit Glyoxal in die entsprechenden Tetrahydroisochinolin-1-carbonsäuren (XI) überführt werden.

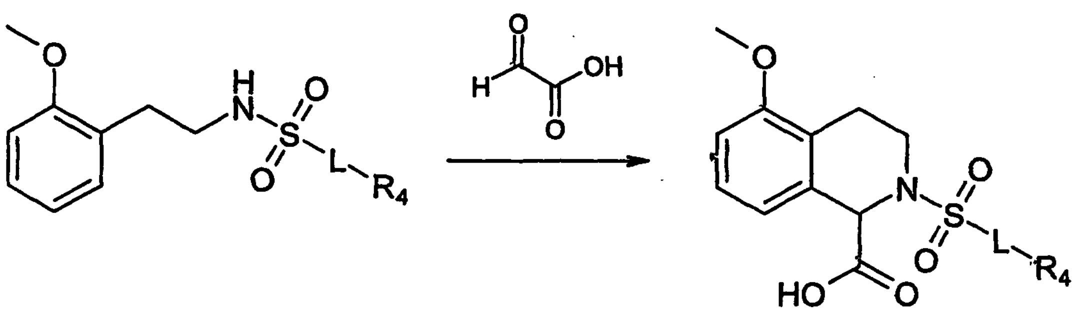

(X) (XI)

Anschließende Etherspaltung durch Bortribromid liefert das freie Phenol (XII), das durch herkömmliche Verfahren in den analogen Ester (XIII) überführt werden kann.

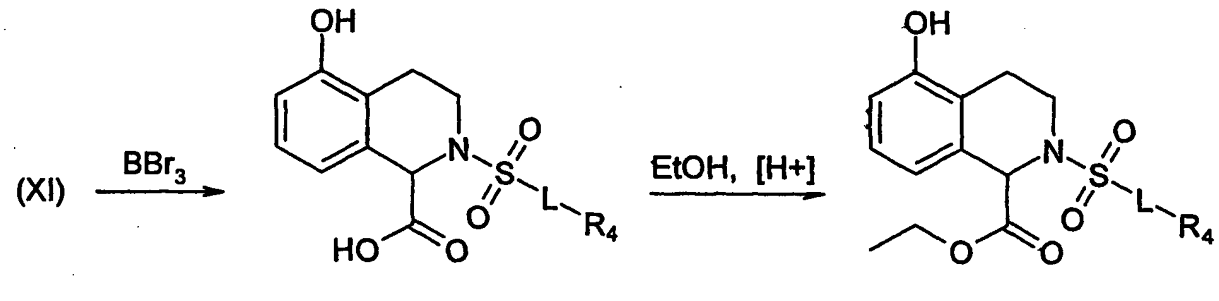


(XII) (XIII)

Verbindung (XIII) wird wiederum analog zu der bei (IV) beschriebenen Mitsunobu-Reaktion in den entsprechenden Ether (XIV) überführt, der nun die hydrophile Seitenkette in der 5-Position des Tetrahydroisochinolinbausteins trägt.

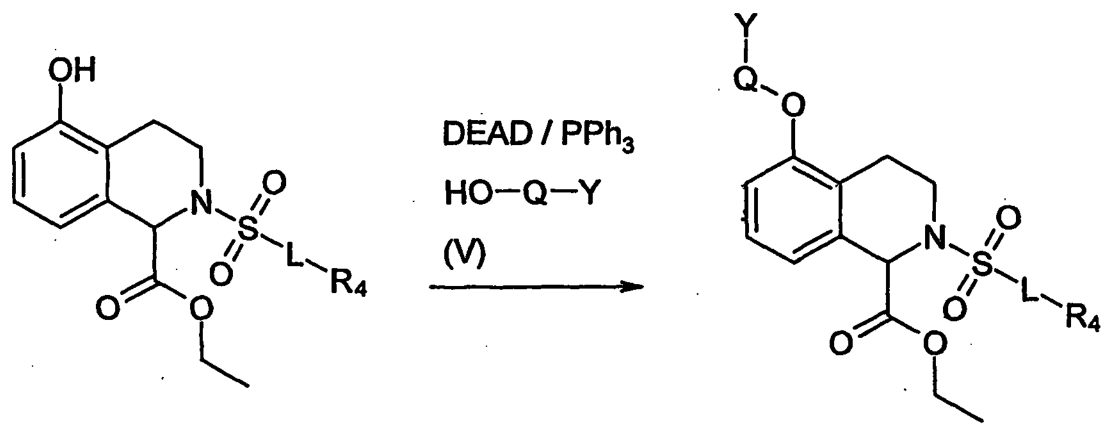


(VIII) (XIV)

Analoges Vorgehen wie bei den 6-substituierten Derivaten liefert nach Verseifung die freien Carbonsäuren (XV), die anschließend in die analogen Hydroxamsäuren (XVI) überführt werden können.

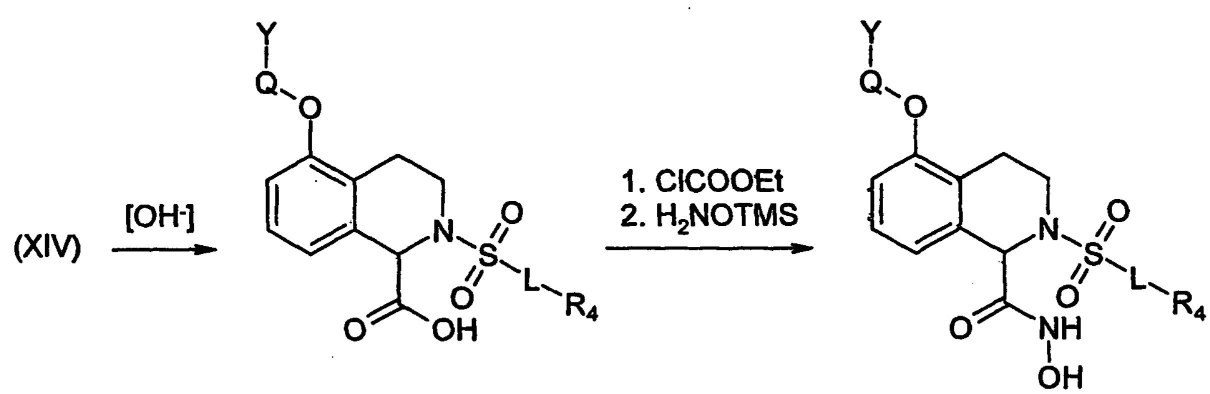


(XV) (XVI)

Alternativ hierzu lassen sich die Zwischenverbindungen X mittels Bortribromid in die entsprechenden Phenole (XVII) spalten, die dann direkt in eine Mitsunobu-Reaktion eingesetzt werden können, um die Alkohole V einzuführen.

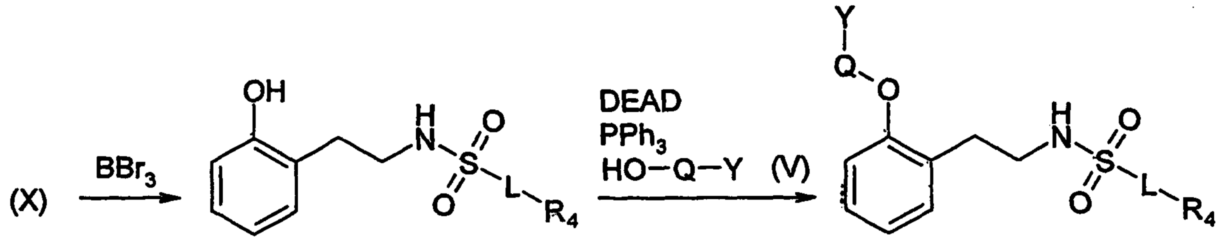


(X) (XVII) (XVIII)

Weitere Umsetzung mit Glyoxal in einer Pictet-Spengler Reaktion liefert die Tetrahydroisochinolin-carbonsäuren (XV), die analog zu den bereits beschriebenen Synthesen in die entsprechenden Hydroxamsäuren (XVI) überführt werden können.

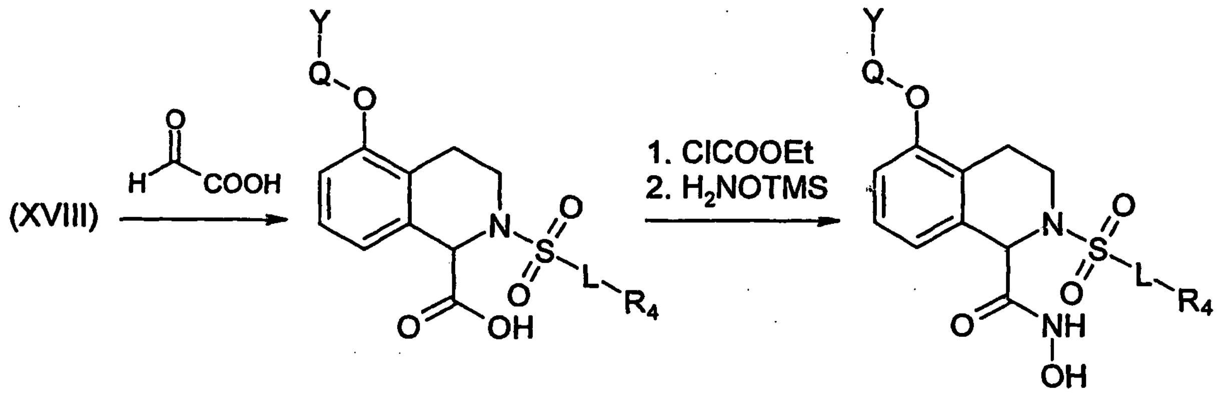


(XVIII) (XV) (XVI)

In Analogie zu dieser Synthese lassen sich auch die Verbindungen mit weiteren Substituenten am Tetrahydroisochinolin-baustein herstellen, in dem man bereits mehrfach substituierte Phenylethylamine (XIX) als Startmaterialien verwendet:

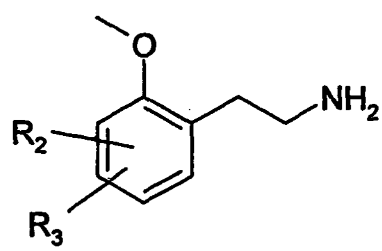

(XIX)

Diese lassen sich analog zu (IX) durch die gleichen Synthesesequenzen in die gewünschten Carbonsäuren (XX), bzw. Hydroxamsäuren (XXI) überführen.

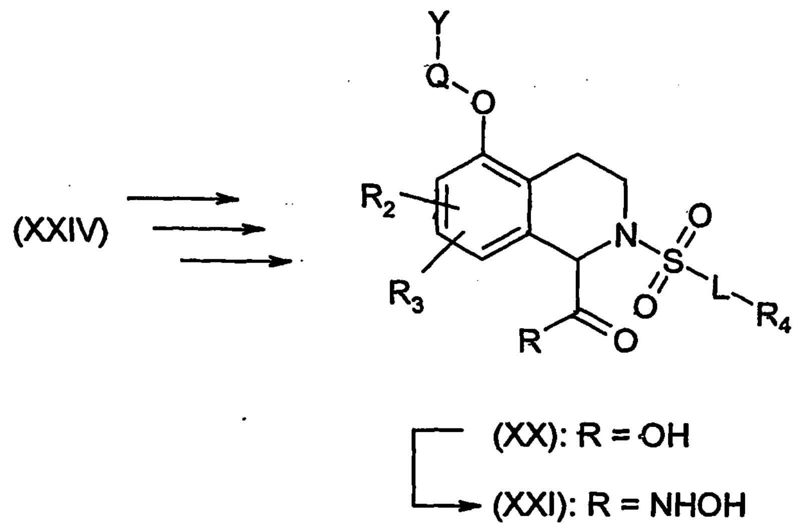


Ausgehend von den gezeigten Synthesen zu den 5-, bzw. 6-substituierten Tetrahydroisochinolin-1-carbonsäuren (XX) bzw. (VII) lassen sich Derivate, wie die entsprechenden Ester, Thiole oder Amide gemäß dem folgenden allgemeinen Syntheseschema herstellen:

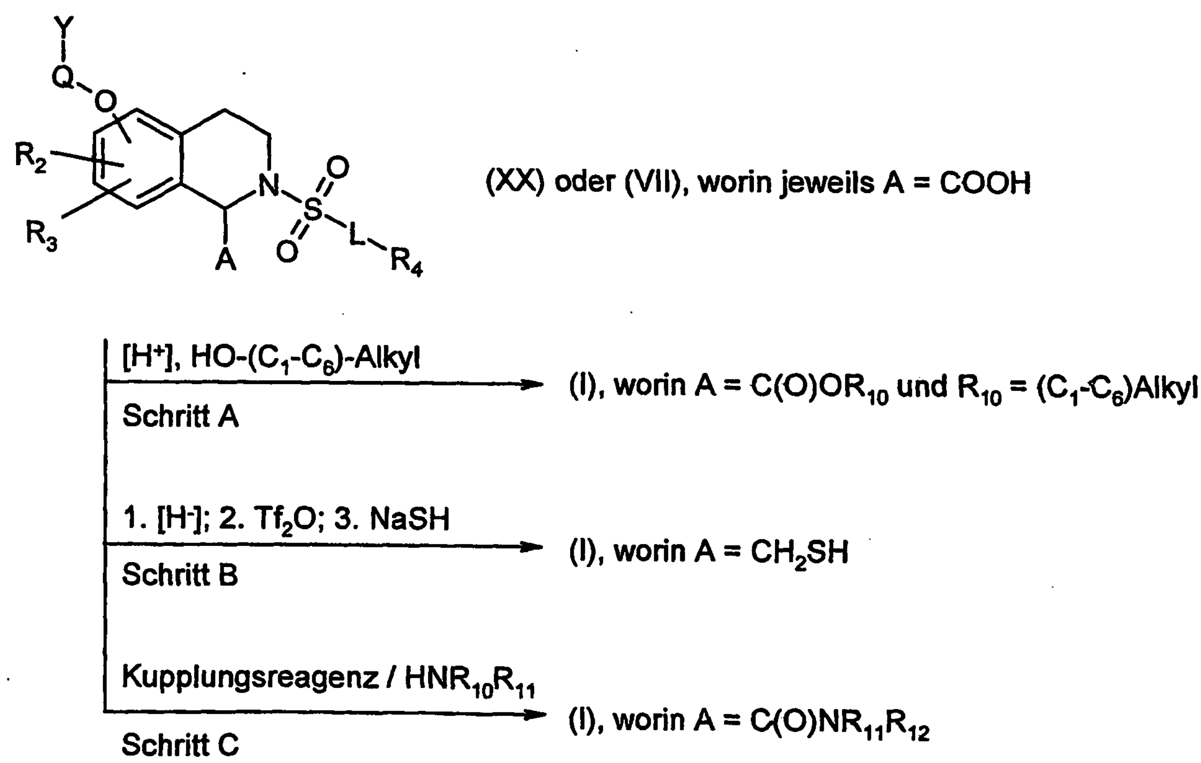


Die dem Fachmann bekannte säurekatalysierte Veresterung -(Schritt A) der Carbonsäuren (XX) bzw. (VII) mit ($C_1$-$C_6$)-Alkylalkoholen liefert Verbindungen der Formel (I), bei denen A gleich C(O)O($C_1$-$C_6$)Alkyl ist.

Reduktion mit beispielsweise $LiAlH_4$, anschließende Überführung des entstandenen Alkohols in eine Abgangsgruppe mit zum Beispiel Trifluormethan-sulfonsäureanhydrid ($Tf_2O$) und nukleophile Substitution mit beispielsweise NaSH (Schritt B) liefert Verbindungen der Formel (I), bei denen A gleich $CH_2SH$ ist.

Ferner lassen sich die Carbonsäuren (XX) oder (VII) auch in Gegenwart von Aminen $HNR_{10}R_{11}$ durch dem Fachmann bekannte Amidkupplungsmethoden, wie z. B. Dicyclohexylcarbodiimid-vermittelt, in Verbindungen der Formel

(I) überführen, bei denen A gleich $C(O)NR_{10}R_{11}$ ist.

Gegenstand der Erfindung ist daher ferner ein Verfahren zur Herstellung einer Verbindung der Formel (I), **dadurch gekennzeichnet dass**

in Schritt 1 ein Tetrahydroisochinolin der Formel (XXII)

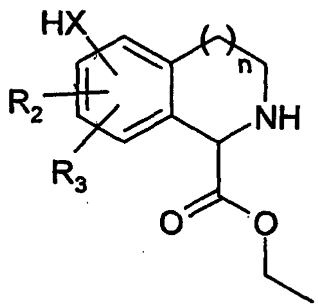

**(XXII)**

mit einem Sulfonsäurechlorid (III) umgesetzt wird,

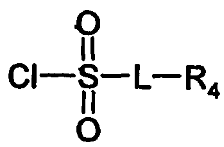

**(III)**

wobei das Sulfonamid (XXIII) gebildet wird

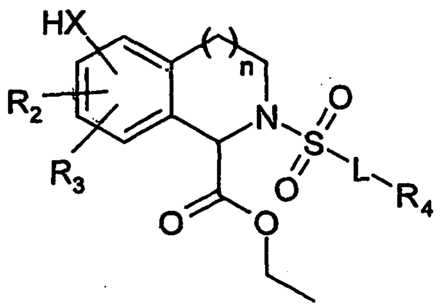

**(XXIII),**

in Schritt 2 die Verbindung (XXIII) mit einem Dialkylazodicarboxylat und Triphenylphosphin und einer Verbindung HO-Q-Y (V) zur Verbindung (XXIV) umgesetzt wird,

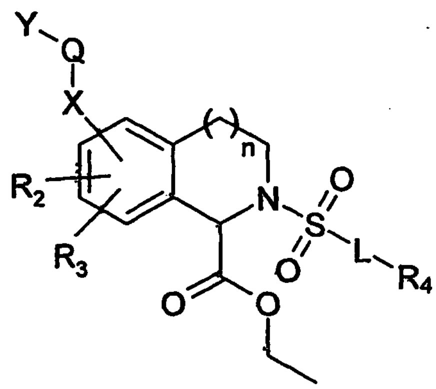

(XXIV),

in Schritt 3 die Estergruppe in der Verbindung (XXIV) mittels einer Base verseift wird, wobei sich eine Verbindung der Formel (I) bildet, in der A O(O)OR$_{10}$ ist und R$_{10}$ gleich H ist,
und optional in Schritt 4 mittels saurer Veresterung durch Umsetzung mit einem (C$_1$-C$_6$)Alkylchlorid eine Verbindung der Formel (I) hergestellt wird, in der A gleich C(O)OR$_{10}$ ist und R$_{10}$ gleich (C$_1$-C$_6$)Alkyl ist,
oder optional in Schritt 5 durch Umsetzung mit Cl-COOEt und anschließender Zugabe von R$_{10}$HNOTMS eine Verbindung der Formel (I) hergestellt wird, in der A gleich C(O)NR$_{10}$OH ist,
oder optional in Schritt 6 ein Verbindung der Formel (I) hergestellt wird, in der A gleich $CH_2SH$ ist, wobei die Carbonsäure zunächst mit einem Hydrid reduziert wird und in einem Folgeschritt der dabei intermediär entstandene Alkohol mit einer Abgangsgruppe versehen wird, die durch anschließende Reaktion mit einem Thiolat ($SH^-$) das entsprechenden Thiol liefert,
oder optional in Schritt 7 ein Verbindung der Formel (I) hergestellt wird, in der A gleich C(O)NR$_{10}$R$_{11}$ ist, wobei die Carbonsäure in Gegenwart einer Base mit einem Amin (C$_1$-C$_6$)Alkyl-NH$_2$ umgesetzt wird.

Es hat sich nun gezeigt, dass die erfindungsgemäßen Verbindungen der allgemeinen Formel (I) mit einer Seitenkette des Typs X-Q-Y eine signifikant bessere Löslichkeit in wässrigen Medien aufweise als die analogen unsubstituierten Verbindungen. Desweiteren wird die Proteinbindung durch Einfügen des Substituenten X-Q-Y verringert. Die Aktivität der Hydroxamsäuren in den serumbasierten Testsystemen konnte damit deutlich verbessert werden. Überraschenderweise wurde ferner gefunden, dass im Falle von Verbindungen der Formel (I), in denen A = COOH ist (Carbonsäuren), eine deutliche Steigerung der Aktivität auftritt. Dies zeigt sich insbesondere in den serumbasierten Testsystemen, in denen Carbonsäuresubstituierte Verbindungen ohne die hier beschriebenen Seitenketten (X-Q-Y) bislang keine Aktivität zeigten.

Tabelle 1: Hydroxamsäure-Derivate

| Nr. | Beispielverbindung | Struktur | MMP-9* [μM | Löslichkeit** [μM] | Proteinbindung (human), [%] | X-Q-Y |
|---|---|---|---|---|---|---|
| 1 | - (Vergleichsverbindung) | 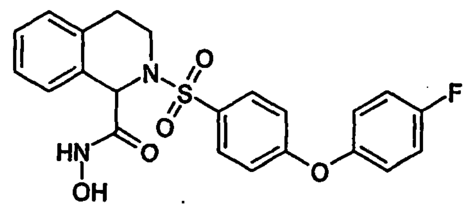 | 2,5 | <1 | 99,8 | Vergleichsverbindung ohne (X-Q-Y)-Seitenkette |
| 2 | **3** | 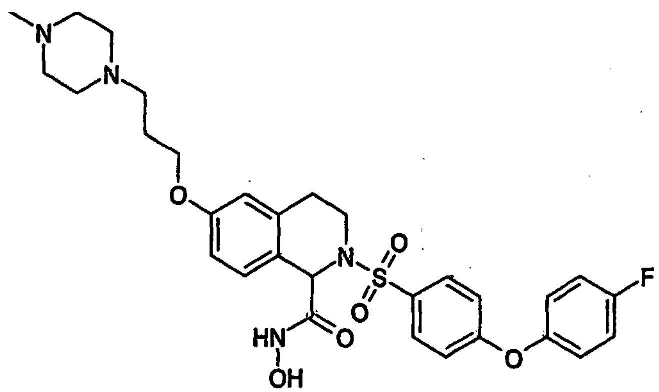 | 0,2 | 23 | 99,0 | 3-(4-Methyl-piperazin-1-yl)-propoxy- |
| 3 | **9** | 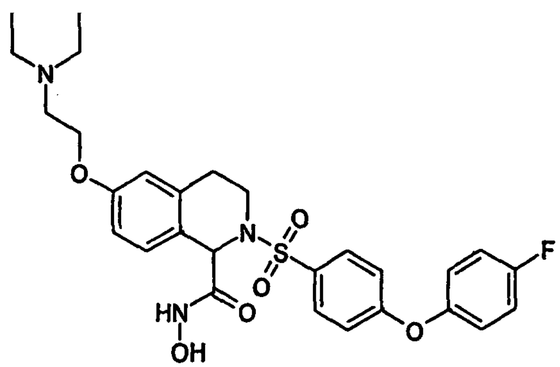 | 0,02 | 153 | 93,9 | 2-Diethylamino-ethoxy- |

(fortgesetzt)

| Nr. | Beispielverbindung | Struktur | MMP-9* [μM | Löslichkeit** [μM] | Proteinbindung (human), [%] | X-Q-Y |
|---|---|---|---|---|---|---|
| 5 | **13** | 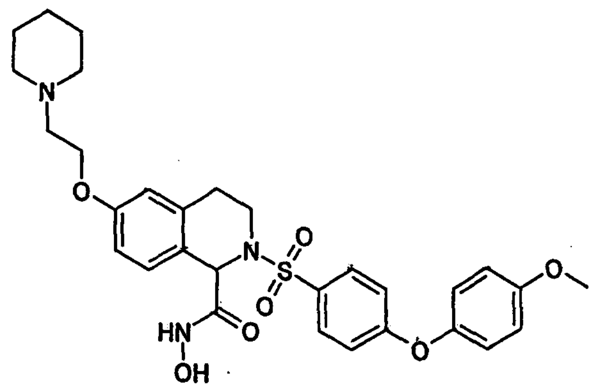 | U,6 | 16 | 98,9 | 2-Piperidin-1-yl-ethoxy- |
| 4 | **15** | 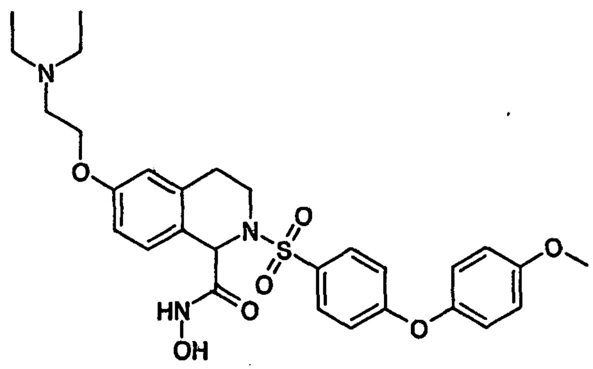 | 0,2 | 107 | 98,8 | 2-Diethylamino-ethoxy- |

* serum basierter Assay; ** Löslichkeitsmessungen mittels CLND (Chemo Lumineszent Nitrogen Detector)

Tabelle 2: Carbonsäure-Derivate

| Nr. | Beispielverbindung | Struktur | MMP-9 Aktivität [µM] | X-Q-Y |
|---|---|---|---|---|
| 1 | - (Vergleichsverbindung) | 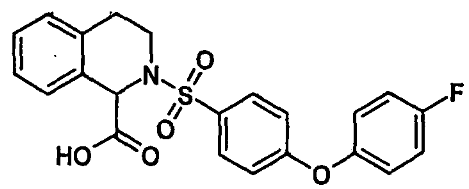 | > 100 | Vergleichsverbindung ohne (X-Q-Y)-Seitenkette |
| 2 | **2** | 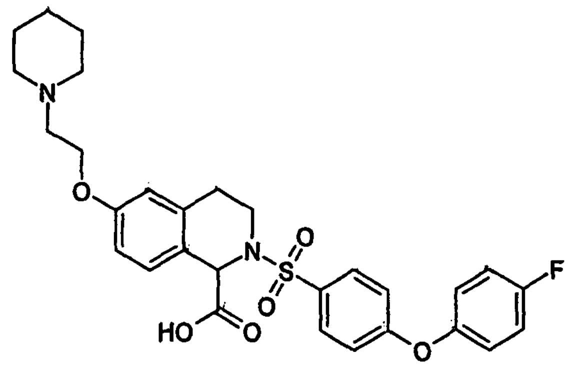 | 8,7 | 2-Piperidin-1-yl-ethoxy- |
| 3 | **14** | 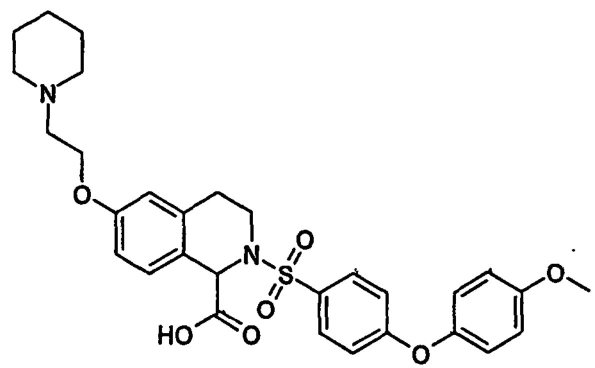 | 7,5 | 2-Piperidin-1-yl-ethoxy- |

(fortgesetzt)

| Nr. | Beispielverbindung | Struktur | MMP-9 Aktivität [µM] | X-Q-Y |
|---|---|---|---|---|
| 4 | **16** | 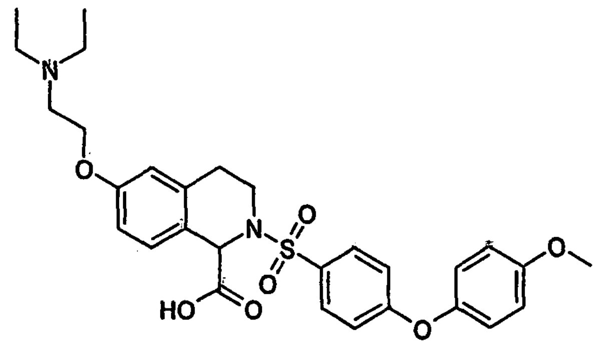 | 6,6 | 2-Diethylamino-ethoxy- |
| * serum basierter Assay | | | | |

Die Erfindung betrifft ferner Arzneimittel beinhaltend einen Gehabt an mindestens einer Verbindung der Formel (I) und/oder eines physiologisch verträglichen Salzes der Verbindung der Formel (I) und/oder eine gegebenenfalls stereoisomere form der Verbindung der Formel (I), zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen zur selektiven Prophylaxe und/oder Therapie all solcher Erkrankungen, an deren Verlauf eine verstärkte Aktivität der Metalloproteinasen beteiligt ist. Dazu gehören die einleitend beschriebenen Indikationen. Insbesondere betrifft die Erfindung die Verwendung einer Verbindung der Formel (I) zur Behandlung von kardiovaskulären Erkrankungen wie Remodelling des Herzens nach einem Herzinfarkt und Atherosklerose oder von instabiler Angina Pectoris, Herzversagen, Stenose, septischer Schock, Entzündungen, Krebserkrankungen, Tumormetastasenbildung, Kachexie, Anorexie, Ulceration, degenerativen Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen, von Erkrankungen des Bindegewebes wie Kollagenosen, Periodontalerkrankungen, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechseis, und zur Prophylaxe von Myokard- und Cerebralinfarkten.

Die Applikation der erfindungsgemäßen Arzneimittel kann durch orale, inhalative, rektale oder transdermale Applikation oder durch subkutane, intraartikuläre, intraperitoneale oder intravenöse Injektion erfolgen. Bevorzugt ist die orale Applikation.

Die Erfindung betrifft auch ein Verfahren zur Herstellung eines Arzneimittels, das **dadurch gekennzeichnet, dass** man mindestens eine Verbindung der Formel (I) mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche physiologisch verträglichen Hilfsmittel- oder Trägerstoffe wie Spreng-, Binde-, Überzugs-, Quellungs-, Gleit- oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuss- oder Sesamöl, Polyethylenglykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind je nach Wirksamkeit der Verbindung gemäß Formel (I), Tagesdosen von etwa 2 mg bis 1000 mg Wirkstoff, bevorzugt etwa 50 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Die erfindungsgemäßen Arzneimittel werden im allgemeinen oral oder parenteral verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierter Wirkstoff-Freigabe, bei deren Herstellung üblicherweise Trägerstoffe und Zusätze und/oder Hilfsmittel wie Spreng-, Binde-, Überzugs-, Quellungs-, Gleit- oder Schmiermittel, Geschmacksstoffe, Süßungsmittel oder Lösungsvermittler Verwendung finden.

Übliche pharmakologisch geeignete Träger- oder Hilfsstoffe sind beispielsweise Magnesiumcarbonat, Titandioxid, Laktose, Mannit und andere Zucker, Talkum, Milcheiweiß, Gelatine, Stärke, Vitamine, Cellulose und ihre Derivate, tierische oder pflanzliche Öle, Polyethylenglykole und Lösungsmittel, wie etwa steriles Wasser, Alkohole, Glycerin und mehrwertige Alkohole.

Gegebenenfalls können die Dosierungseinheiten für die orale Verabreichung mikroverkapselt werden, um die Abgabe zu verzögern oder über einen längeren Zeitraum auszudehnen, wie beispielsweise durch Überziehen oder Einbetten des Wirkstoffs in Teilchenform in geeignete Polymere, Wachse oder dergleichen.

Vorzugsweise werden die pharmazeutischen Präparate in Dosierungseinheiten hergestellt und verabreicht, wobei jede Einheit als aktiven Bestandteil eine bestimmte Dosis einer oder mehrerer Verbindungen der erfindungsgemäßen Spirobenzofuranlactam-Derivate enthält. Bei festen Dosierungseinheiten wie Tabletten, Kapseln und Suppositorien kann diese Dosis bis zu etwa 500 mg, bevorzugt jedoch etwa 0,1 bis 200 mg, und bei Injektionslösungen in Ampullenform bis zu etwa 200 mg, vorzugsweise aber etwa 0,5 bis 100 mg, pro Tag betragen.

Die zu verabreichende Tagesdosis ist abhängig vom Körpergewicht, Alter, Geschlecht und Zustand des Säugers. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber in mehreren kleineren Dosierungseinheiten als auch durch Mehrfachgabe unterteilter Dosen in bestimmten Intervallen erfolgen.

Die erfindungsgemäßen Arzneimittel werden dadurch hergestellt, dass man ein oder mehrere der erfindungsgemäßen Verbindungen der Formel (I) mit einem oder mehreren der üblichen Träger- oder Hilfsstoffe in eine geeignete Dameichungsform bringt.

In den sich anschließenden Beispielen wird die Erfindung weiter erläutert. Prozentangaben beziehen sich auf das Gewicht. Mischungsverhältnisse bei Flüssigkeiten beziehen sich auf das Volumen, wenn keine anderen Angaben gemacht wurden.

Experimenteller Teil

Liste der verwendeten Abkürzungen:

| | |
|---|---|
| abs. | absolut |
| ACN | Acetonitril |
| Bsp. | Beispiel |
| DEAD | Diethylazodicarboxylat |
| DMF | Dimethylformamid |
| eq. | Äquivalent |
| ESI | Electro Spray Ionization |
| konz. | konzentriert |
| LCMS | Liquid Chromatography Mass Spectrometry |
| Merck | Firma Merck KGaA, Deutschland |
| Rt | Retentionszeit |
| TFA | Trifluoro acetic acid; Trifluoressigsäure |
| THF | Tetrahydrofuran |
| YMC | Firma YMC, Japan |
| Vbdg. | Verbindung |

Beispiel 1: Herstellung von Beispielverbindungen

**Beispielverbindungen 3 und 4:**

**3:** 2-[4-(4-Fluor-phenoxy)-benzolsulfonyl]-6-[3-(4-methyl-piperazin-1-yl)-propoxy]-1,2,3,4-tetrahydro-isochinolin-1-carbonsäure-hydroxamid;
**4:** 2-[4-(4-Fluor-phenoxy)-benzolsulfonyl]-6-[3-(4-methyl-piperazin-1-yl)-propoxy]-1,2,3,4-tetrahydro-isochinolin-1-carbonsäure;

**3A:** 4-Fluorphenoxy-benzolsulfonsäurechlorid;

Zu einer auf 0 ˚C gekühlten Lösung von 13,05 g 4-Fluorphenoxybenzol (0,069 mol) werden 16,1 g (0,138 mol) Chlorsulfonsäure langsam zugetropft. Nach beendeter Zugabe wird für weitere 4 Stunden bei Raumtemperatur gerührt. Zur Aufarbeitung werden 50 ml Dichlormethan zugegeben und einmal mit $H_2O$ gewaschen. Die wässrige Phase wird abgetrennt und noch zweimal mit je 50 ml Dichlormethan extrahiert. Die vereinigten organischen Phasen werden mit $MgSO_4$ getrocknet und eingeengt, wobei die Titelverbindung als farbloser Feststoff erhalten wird. Ausbeute: 11,03 g; 60%.

**3B:** 2-[4-(4-Fluor-phenoxy)-benzolsulfonyl]-6-hydroxy-1,2,3,4-tetrahydro-isochinolin-1-carbonsäure-methylester;

Eine Lösung von 360 mg (1,7 mmol) 6-Hydroxy-tetrahydroisochinolin-1-carbonsäuremethylester-hydrochlorid in 5 ml Pyridin wird bei 0 ˚C mit 512 mg (1,95 mmol) 4-Fluorphenoxy-benzolsulfonsäurechlorid versetzt. Die Lösung wird 18 Stunden bei Raumtemperatur gerührt und im Anschluß vom Lösungsmittel befreit. Der Rückstand wird in Essigester aufgenommen und dreimal mit gesättigter $NH_4Cl$-Lösung gewaschen, mit $MgSO_4$ getrocknet und eingeengt, wobei die Titelverbindung als gelber Feststoff isoliert werden kann, Ausbeute 98%.

**3C:** 2-[4-(4-Fluor-phenoxy)-benzolsulfonyl]-6-[3-(4-methyl-piperazin-1-yl)-propoxy]-1,2,3,4- tetrahydro-isochinolin-1-carbonsäure-methylester;

250 mg (0,55 mmol) 2-[4-(4-Fluor-phenoxy)-benzolsulfonyl]-6-hydroxy-1,2,3,4-tetrahydro-isochinolin-1-carborrsäure-methylester werden in 5 ml THF gelöst. Bei Raumtemperatur werden 216 mg (0,825 mmol) Triphenylphosphin, 142 µl (0,825 mmol) DEAD, sowie 120 mg (0,825 mmol) 3-(4-Methyl-piperazin-1-yl)-propan-1-ol zugegeben und die Lösung 18 Stunden bei Raumtemperatur gerührt. Im Anschluß wird vom Lösungsmittel befreit und der Rückstand an Kieselgel chromatographiert, wobei das gewünschte Produkt als farbloser Feststoff isoliert werden kann. Ausbeute 76%.

**3:** 2-[4-(4-Fluor-phenoxy)-benzolsulfonyl]-6-[3-(4-methyl-piperazin-1-yl)-propoxy]-1,2,3,4-tetrahydro-isochinolin-1-carbonsäure -hydroxamid;

**4:** 2-[4-(4-Fluor-phenoxy)-benzolsulfonyl]-6-[3-(4-methyl-piperazin-1-yl)-propoxy]-1,2,3,4-tetrahydro-isochinolin-1-carbonsäure;

Eine Lösung von 61 mg (0,88 mmol) Hydroxylamin-hydrochlorid in 1 ml $H_2O$ wird mit einer Lösung von 56 mg (1,4 mmol) NaOH in 0,5 ml $H_2O$ versetzt und für 10 Minuten gerührt. Bei 0 ˚C wird eine Lösung von 240 mg (0,4 mmol) 2-[4-(4-Fluor-phenoxy)-benzolsulfonyl]-6-[3-(4-methyl-piperazin-1-yl)-propoxy]-1,2,3,4- tetrahydro-isochinolin-1-carbonsäure-methylester in 0,5 ml Dioxan zugegeben und bei 0 ˚C gerührt. Zum Erreichen eines vollständigen Umsatzes wird noch zweimal mit 61 mg (0,88 mmol) Hydroxylamin-hydrochlorid, sowie 56 mg (1,4 mmol) NaOH versetzt. Zur Aufarbeitung wird mit 1 N HCl neutralisiert und mit Essigester extrahiert. Die organische Phase wird abgetrennt, mit $MgSO_4$ getrocknet und eingeengt. Chromatohraphie des Rückstandes an Kieselgel liefert die Hydroxamsäure (Beispielverbindung 3) als farblosen Feststoff in 46% Ausbeute, sowie die Carbonsäure (Beispielverbindung 4), ebenfalls als farblosen Feststoff in einer Ausbeute von 15%.

**Beispielverbindungen 1 und 2:**

**1:** 2-[4-(4-Fluor-phenoxy)-benzolsulfonyl]-6-(2-piperidin-1-yl-ethoxy)-1,2,3,4-tetrahydro-isochinolin-1-carbonsäure-hydroxamid;

**2:** 2-[4-(4-Fluor-phenoxy)-benzolsulfonyl]-6-(2-piperidin-1-yl-ethoxy)-1,2,3,4-tetrahydro-isochinolin-1-carbonsäure;

**1A:** 2-[4-(4-Fluor-phenoxy)-benzolsulfonyl]-6-(2-piperidin-1-yl-ethoxy)-1,2,3,4-tetrahydro-isochinolin-1-carbonsäure-methylester;

2-[4-(4-Fluor-phenoxy)-benzolsulfonyl]-6-hydroxy-1,2,3,4-tetrahydro-isochinolin-1-carbonsäure-methylester (3B) wird nach der unter 3C beschriebenen Methode mit 2-Piperidin-4-yl-ethanol umgesetzt.

**1:** 2-[4-(4-Fluor-phenoxy)-benzolsulfonyl]-6-(2-piperidin-1-yl-ethoxy)-1,2,3,4-tetrahydro-isochinolin-1-carbonsäure-hydroxamid;

**2:** 2-[4-(4-Fluor-phenoxy)-benzolsulfonyl]-6-(2-piperidin-1-yl-ethoxy)-1,2,3,4-tetrahydro-isochinolin-1-carbonsäure;

Die Titelverbindungen werden ausgehend von 2-[4-(4-Fluor-phenoxy)-benzolsulfonyl]-6-(2-piperidin-1-yl-ethoxy)-1,2,3,4-tetrahydro-isochinolin-1-carbonsäure-methylester nach der unter Beispielverbindungen 3/4 beschriebenen Methode hergestellt.

**Beispielverbindungen 5 und 6:**

**5:** 2-[4-(4-Fluor-phenoxy)-benzolsulfonyl]-6-(2-methoxy-ethoxy)-1,2,3,4-tetrahydro-isochinolin-1-carbonsäure-hydroxyamid;

**6:** 2-[4-(4-Fluor-phenoxy)-benzolsulfonyl]-6-(2-methoxy-ethoxy)-1,2,3,4-tetrahydro-isochinolin-1-carbonsäure;

**5A:** 2-[4-(4-Fluor-phenoxy)-benzolsulfonyl]-6-(2-methoxy-ethoxy)-1,2,3,4-tetrahydro-isochinolin-1-carbonsäure-methylester;

2-[4-(4-Fluor-phenoxy)-benzolsulfonyl]-6-hydroxy-1,2,3,4-tetrahydro-isochinolin-1-carbonsäure-methylester (3B) wird nach der unter 3C beschriebenen Methode mit 2-Methoxy-ethanol umgesetzt.

**5:** 2-[4-(4-Fluor-phenoxy)-benzolsulfonyl]-6-(2-methoxy-ethoxy)-1,2,3,4-tetrahydro-isochinolin-1-carbonsäure-hydroxyamid;

**6:** 2-[4-(4-Fluor-phenoxy)-benzolsulfonyl]-6-(2-methoxy-ethoxy)-1,2,3,4-tetrahydro-isochinolin-1-carbonsäure;

Die Titelverbindungen werden ausgehend von 2-[4-(4-Fluor-phenoxy)-benzolsulfonyl]-6-(2-methoxy-ethoxy)-1,2,3,4-tetrahydro-isochinolin-1-orbonsäure-methylester nach der unter Beispielverbindungen 3/4 beschriebenen Methode hergestellt.

**Beispielverbindungen 7 und 8:**

**7:** 2-[4-(4-Fluor-phenoxy)-benzolsulfonyl]-6-(2-morpholin-4-yl-ethoxy)-1,2,3,4-tetrahydro-isochinolin-1-carbonsäure-hydroxyamid;

**8:** 2-[4-(4-Fluor-phenoxy)-benzolsulfonyl]-6-(2-morpholin-4-yl-ethoxy)-1,2,3,4-tetrahydro-isochinolin-1-carbonsäure;

**7A:** 2-[4-(4-Fluor-phenoxy)-benzolsulfonyl]-6-(2-morpholin-4-yl-ethoxy)-1,2,3,4-tetrahydro-isochinolin-1-carbonsäure-methylester;
667 mg (2,0 mmol) polymergebundenes $PPh_3$ (3 mmol/g) wird in 2 ml THF vorgelegt und bei Raumtemperatur mit 229 mg (0,5 mmol) 2-[4-(4-Fluoro-phenoxy)-benzolsulfonyl]-6-hydroxy-1,2,3,4-tetrahydro-isochinolin-1-carbonsäure-methylester (3B), 242 $\mu$l (2,0 mmol) 2-Morpholin-4-yl-ethanol und 311 $\mu$l (2,0 mmol) DEAD versetzt. Nach einer Stunde wird abfiltriert und das Filtrat i. Vak. eingeengt. Der Rückstand wird an Kieselgel chromatographiert (Dichlormethan $\rightarrow$ Dichlormethan / Methanol 98:2), wobei 53% der Titelverbindung erhalten werden.

**8:** 2-[4-(4-Fluor-phenoxy)-benzolsulfonyl]-6-(2-morpholin-4-yl-ethoxy)-1,2,3,4-tetrahydro-isochinolin-1-carbonsäure;
86 mg (0,15 mmol) 2-[4-(4-Fluor-phenoxy)-benzolsulfonyl]-6-(2-morpholin-4-yl-ethoxy)-1,2,3,4-tetrahydro-isochinolin-l-carbonsäure-methylester werden in 15 ml 6 N HCl auf 70 ˚C erhitzt. Nach einer Stunde wird auf Raumtemperatur abkühlen lassen und mit 5 N NaOH neutralisiert. Die wässrige Lösung wird mit Essigester extrahiert, die organische Phase mit $MgSO_4$ getrocknet und eingeengt, wobei die Carbonsäure in einer Ausbeute von 75% isoliert werden kann.

**7B:** 2-[4-(4-Fluor-phenoxy)-benzolsulfonyl]-6-(2-morpholin-4-ylethoxy)-1,2,3,4-tetrahydro-isochinolin-1-carbonsäure-(tetrahydro-pyran-2-yloxy)-amid;
Zu einer Lösung von 98 mg (0,176 mmol) 2-[4-(4-Fluor-phenoxy)-benzotsuifonyl]-6-(2-morpholin-4-yl-ethoxy)-1,2,3,4-tetrahydro-isochinolin-1-carbonsäure und 46 mg (0,325 mmol) O-(Tetrahydro-pyran-2-yl)-hydroxylamin in 3 ml DMF wurde bei 0 ˚C eine Lösung von 40 mg (0,264 mmol) HOBt und 51 mg (0,264 mmol) EDC in 2 ml DMF zugetropft und bei Raumtemperatur gerührt. Nach Stehen über Nacht wurde auf $H_2O$ gegeben und mit Essigester extrahiert. Die organische Phase wurde mit $MgSO_4$ getrocknet und eingeengt. Der Rückstand wurde an Kieselgel chromatographiert (Dichlormethan $\rightarrow$ Dichlormethan / Methanol 99:1), wobei dir Titelverbindung als farbloser Feststoff isoliert wurde. Ausbeute: 80%.

**7:** 2-[4-(4-Fluor-phenoxy)-benzolsulfonyl]-6-(2-morpholin-4-yl-ethoxy)-1,2,3,4-tetrahydro-isochinolin-1-carbonsäure-hydroxyamid;
93 mg (0,141 mmol) 2-[4-(4-Fluor-phenoxy)-benzolsulfonyl]-6-(2-morpholin-4-yl-ethoxy)-1,2,3,4-tetrahydro-isochinolin-1-carbonsäure-(tetrahydro-pyran-2-yloxy)-amid wurden in 5 ml Metahnol mit 40 mg (0,212 mmol) p-Toluolsulfonsäure versetzt und bei Raumtemperatur gerührt. Nach drei Stunden wurde vom Lösungsmittel berfreit und der Rückstand zwischen $H_2O$ und Essigester verteilt. Die organische Phase wurde isoliert, mit $MgSO_4$ getrocknet und eingeengt. Der Rückstand wurde an Kieselgel chromatographiert (Dichlormethan $\rightarrow$ Dichlormethan / Methanol 1:1), wobei die Titelverbindung nach Verreiben mit Diisopropylether als farboser Feststoff isoliert werden konnte. Ausbeute 25%.

**Beispielverbindungen 9 und 10:**

**9:** 6-(2-Diethylamino-ethoxy)-2-[4-(4-fluor-phenoxy)-benzolsulfonyl]-1,2,3,4-tetrahydro-isochinolin-1-carbonsäure-hydroxyamid;

**10:** 6-(2-Diethylamino-ethoxy)-2-[4-(4-fluor-phenoxy)-benzolsulfonyl]-1,2,3,4-tetrahydro-isochinolin-1-carbonsäure;

**9A:** 6-(2-Diethylamino-ethoxy)-2-[4-(4-fluor-phenoxy)-benzolsulfonyl]-1,2,3,4-tetrahydro-isochinolin-1-carbonsäure-methylester;
2-[4-(4-Fluor-phenoxy)-benzolsulfonyl]-6-hydroxy-1,2,3,4-tetrahydro-isochinolin-1-carbonsäure-methylester (3B)

wird nach der unter 3C beschriebenen Methode mit 2-Diethylamino-ethanol umgesetzt.

**10:** 6-(2-Diethylamino-ethoxy)-2-[4-(4-fluor-phenoxy)-benzolsulfonyl]-1,2,3,4-tetrahydro-isochinolin-1-carbonsäure; 6-(2-Diethylamino-ethoxy)-2-[4-(4-fluor-phenoxy)-benzolsulfonyl]-1,2,3,4-tetrahydro-isochinolin-1-carbonsäure-methylester wird analog der unter 8 beschriebenen Methode sauer verseift.

**9:** 6-(2-Diethylamino-ethoxy)-2-[4-(4-fluor-phenoxy)-benzolsulfonyl]-1,2,3,4-tetrahydro-isochinolin-1-carbonsäure-hydroxyamid; 6-(2-Diethylamino-ethoxy)-2-[4-(4-fluor-phenoxy)-benzolsulfonyl]-1,2,3,4-tetrahydro-isochinolin-1-carbonsäure wird nach der unter 19 beschriebenen Methode in die gewünschte Hydroxamsäure überführt.

**Beispielverbindungen 11 und 12:**

**11:** 2-[4-(4-Fluor-phenoxy)-benzolsulfonyl]-6-(2-imidazol-1-yl-ethoxy)-1,2,3,4-tetrahydro-isochinolin-1-carbonsäure-hydroxyamid;

**12:** 2-[4-(4-Fluor-phenoxy)-benzolsulfonyl]-6-(2-imidazol-1-yl-ethoxy)-1,2,3,4-tetrahydro-isochinolin-1-carbonsäure;

**11A:** 2-[4-(4-fluor-phenoxy)-benzolsulfonyl]-6-(2-imidazol-1-yl-ethoxy)-1,2,3,4-tetrahydro-isochinolin-1-carbonsäure-methylester; 2-[4-(4-Fluor-phenoxy)-benzolsulfonyl]-6-hydroxy-1,2,3,4-tetrahydro-isochinolin-1-carbonsäure-methylester (3B) wird nach der unter 3C beschriebene Methode mit 2-Imidazol-1-yl-ethanol umgesetzt.

**12:** 2-[4-(4-Fluor-phenoxy)-benzolsulfonyl]-6-(2-imidazol-1-yl-ethoxy)-1,2,3,4-tetrahydro-isochinolin-1-carbonsäure; 2-[4-(4-Fluor-phenoxy)-benzolsulfonyl]-6-(2-imidazol-1-yl-ethoxy)-1,2,3,4-tetrahydro-isochinolin-1-carbonsäure-methylester wird analog der unter 8 beschriebenen Methode sauer verseift.

**11:** 2-[4-(4-Fluor-phenoxy)-benzolsulfonyl]-6-(2-imidazol-1-yl-ethoxy)-1,2,3,4-tetrahydro-isochinolin-1-carbonsäure-hydroxyamid; 2-[4-(4-Fluor-phenoxy)-benzolsulfonyl]-6-(2-imidazol-1-yl-ethoxy)-1,2,3,4-tetrahydro-isochinolin-1-carbonsäure wird nach der unter 19 beschriebenen Methode in die gewünschte Hydroxamsäure überführt.

**Beispielverbindungen 13 und 14:**

**13:** 2-[4-(4-Methoxy-phenoxy)-benzolsulfonyl]-6-(2-piperidin-1-yl-ethoxy)-1,2,3,4-tetrahydro-isochinolin-1-carbonsäure-hydroxyamid;

**14:** 2-[4-(4-Methoxy-phenoxy)-benzolsulfonyl]-6-(2-piperidin-1-yl-ethoxy)-1,2,3,4-tetrahydro-isochinolin-1-carbonsäure;

**13A:** 4-(4-Methoxy-phenoxy)-benzol-sulfonylchlorid; Die Titelverbindung wird nach literaturbekanntem Verfahren durch Chlorsulfonierung von 4-Methoxy-biphenylether hergestellt (Patent US6153757).

**13B:** 6-Hydroxy-2-[4-(4-methoxy-phenoxy)-benzolsulfonyl]-1,2,3,4-tetrahydro-isochinolin-1-carbonsäure-methylester; 4-(4-Methoxy-phenoxy)-benzol-sulfonylchlorid wird analog zu der in 3B beschriebenen Methode mit 6-Hydroxy-tetrahydroisochinolin-1-carbonsäure-methylester-hydrochlorid zu dem gewünschten Sulfonamid umgesetzt.

**13C:** 2-[4-(4-Methoxy-phenoxy)-benzolsulfonyl]-6-(2-piperidin-1-yl-ethoxy)-1,2,3,4-tetrahydro-isochinolin-1-carbonsäure-methylester;

6-Hydroxy-2-[4-(4-methoxy-phenoxy)-benzolsulfonyl]-1,2,3,4-tetrahydro-isochinolin-1-carbonsäure-methylester wird analog zu der unter 3C beschriebenen Methode mit 2-Piperidin-1-yl-ethanol umgesetzt.

**13:** 2-[4-(4-Methoxy-phenoxy)-benzolsulfonyl]-6-(2-piperidin-1-yl-ethoxy)-1,2,3,4-tetrahydro-isochinolin-1-carbonsäure-hydroxyamid;

**14:** 2-[4-(4-Methoxy-phenoxy)-benzotsulfonyl]-6-(2-piperidin-1-yl-ethoxy)-1,2,3,4-tetrahydro-isochinolin-1-carbonsäure;
Die Titelverbindungen werden ausgehend von 2-[4-(4-Methoxy-phenoxy)-benzol-sulfonyl]-6-(2-piperidin-1-yl-ethoxy)-1,2,3,4-tetrahydro-isochinolin-1-carbonsäuremethylester nach der unter Beispielverbindungen 3/4 beschriebenen Methode hergestellt.

**Beispielverbindungen 15 und 16:**

**15:** 6-(2-Diethylamino-ethoxy)-2-[4-(4-methoxy-phenoxy)-benzolsulfonyl]-1,2,3,4-tetrahydro-isochinolin-1-carbonsäure-hydroxyamid;
**16:** 6-(2-Diethylamino-ethoxy)-2-[4-(4-methoxy-phenoxy)-benzolsulfonyl]-1,2,3,4-tetrahydro-isochinolin-1-carbonsäure;
**15A:** 6-(2-Diethylamino-ethoxy)-2-[4-(4-methoxy-phenoxy)-benzolsulfonyl]-1,2,3,4-tetrahydro-isochinolin-1-carbonsäure-methylester;
6-Hydroxy-2-[4-(4-methoxy-phenoxy)-benzolsulfonyl]-1,2,3,4-tetrahydro-isochinolin-1-carbonsäure-methylester (13B) wird analog zu der unter 3C beschriebenen Methode mit 2-Diethylamino-ethanol umgesetzt.
**15:** 6-(2-Diethylamino-ethoxy)-2-[4-(4-methoxy-phenoxy)-benzolsulfonyl]-1,2,3,4-tetrahydro-isochinolin-1-carbonsäure-hydroxyamid;
**16:** 6-(2-Diethylamino-ethoxy)-2-[4-(4-methoxy-phenoxy)-benzolsulfionyl]-1,2,3,4-tetrahydro-isochinolin-1-carbonsäure;
Die Titelverbindungen werden ausgehend von 6-(2-Diethylamino-ethoxy)-2-[4-(4-methoxy-phenoxy)-benzolsulfonyl]-1,2,3,4-tetrahydro-isochinolin-1-carbonsäuremethylester nach der unter Beispielverbindungen 3/4 beschriebenen Methode hergestellt.

**Beispielverbindungen 17 und 18:**

**17:** 6-(2-Imidazol-1-yl-ethoxy)-2-[4-(4-methoxy-phenoxy)-benzolsulfonyl]-1,2,3,4-tetrahydro-isochinolin-1-carbonsäure-hydroxyamid;
**18:** 6-(2-Imidazol-1-yl-ethoxy)-2-[4-(4-methoxy-phenoxy)-benzolsulfonyl]-1,2,3,4-tetrahydro-isochinolin-1-carbonsäure;
**17A:** 6-(2-Imidazol-1-yl-ethoxy)-2-[4-(4-methoxy-phenoxy)-benzolsulfonyl]-1,2,3,4-tetrahydro-isochinolin-1-carbonsäure-methylester;
6-Hydroxy-2-[4-(4-methoxy-phenoxy)-benzolsulfonyl]-1,2,3,4-tetrahydro-isochinolin-1-carbonsäure-methylester (13B) wird analog zu der unter 3C beschriebenen Methode mit 2-Imidazol-1-yl-ethanol umgesetzt.
**17**: 6-(2-Imidazol-1-yl-ethoxy)-2-[4-(4-methoxy-phenoxy)-benzolsulfonyl]-1,2,3,4-tetrahydro-isochinolin-1-carbonsäure-hydroxyamid;
**18:** 6-(2-Imidazol-1-yl-ethoxy)-2-[4-(4-methoxy-phenoxy)-benzolsulfonyl]-1,2,3,4-tetrahydro-isochinolin-1-carbonsäure;
Die Titelverbindungen werden ausgehend von 6-(2-Imidazol-1-yl-ethoxy)-2-[4-(4-methoxy-phenoxy)-benzolsulfonyl]-1,2,3,4-tetrahydro-isochinolin-1-carbonsäure nach der unter Beispielverbindungen 3/4 beschriebenen Methode hergestellt.

**Beispielverbindungen 19 und 20:**

**19:** 2-[4-(4-Fluor-phenoxy)-benzolsulfonyl]-5-(2-piperidin-1-yl-ethoxy)-1,2,3,4-tetrahydro-isochinolin-1-carbonsäure-hydroxyamid;

**20:** 2-[4-(4-Fluor-phenoxy)-benzolsulfonyl]-5-(2-piperidin-1-yl-ethoxy)-1,2,3,4-tetrahydro-isochinolin-1-carbonsäure;

**19A:** 4-(4-Fluor-phenoxy)-N-[2-(2-methoxy-phenyl)-ethyl]-benzolsulfonamid;
0,256 ml (1,74 mmol) ortho-Methoxy-phenethylamin, sowie 0,243 ml (1,74 mmol) Triethylamin werden in 20 ml THF gelöst und bei Raumtemperatur mit einer Lösung aus 500 mg (1,74 mmol) 4-Fluorphenoxy-benzolsulfonsäurechlorid (3A) in 10 ml THF versetzt. Nachdem eine Stunde bei Raumtemperatur gerührt wurde, wird von den entstandenen Salzen abfiltriert und das Filtrat i. Vak. eingeengt, wobei die Titelverbindung in einer Ausbeute von 89% isoliert werden kann.

**19B**: 4-(4-Fluor-phenoxy)-N-[2-(2-hydroxy-phenyl)-ethyl]-benzolsulfonamid;
620 mg (1,54 mmol) 4-(4-Fluor-phenoxy)-N-[2-(2-methoxy-phenyl)-ethyl]-benzolsulfonamid werden in 10 ml Dichlormethan gelöst und bei -40 ˚C mit 3,09 ml (3,09 mmol) einer 1 M Lösung von Bortribromid in Dichlormethan versetzt. Die Lösung wird auf Raumtemperatur erwärmt und weitere vier Stunden gerührt. Im Anschluß wird auf Wasser gegeben, die Phasen getrennt und die organische Phase noch zweimal mit $H_2O$ gewaschen. Die Dichlormethanphase wird mit $MgSO_4$ getrocknet und einrotiert, wobei das gewünschte Phenol als braunes Öl isoliert werden kann. Rohausbeute 89%.

**19C:** 4-(4-Fluor-phenoxy)-N-{2-[2-(2-piperidin-1-yl-ethoxy)-phenyl]-ethyl}-benzolsulfonamid;
200 mg (0,516 mmol) 4-(4-Fluor-phenoxy)-N-[2-(2-hydroxy-phenyl)-ethyl]-benzolsulfonamid werden in 20 ml THF mit 230 mg (0,878 mmol) Triphenylphosphin, 141 µl (0,774 mmol) DEAD und 0,165 ml (1,239 mmol) 2-Piperidin-1-yl-ethahol analog zu der unter 3C beschriebenen Methode umgesetzt, wobei die Titelver-bindung in einer Ausbeute von 69% erhalten wird.

**20:** 2-[4-(4-Fluoro-phenoxy)-benzolsulfonyl]-5-(2-piperidin-1-yl-ethoxy)-1,2,3,4-tetrahydro-isochinolin-1-carbonsäure;
110 mg (0,221 mmol) 4-(4-Fluor-phenoxy)-N-{2-[2-(2-piperidin-1-yl-ethoxy)-phenyl]-ethyl}-benzolsulfonamid werden zusammen mit 49,1 µl (0,441 mmol) Glyoxylsäure (50%ig in $H_2O$) in 5 ml Trifluoressigsäure bei Raumtemperatur gerührt. Nach Stehen über Nacht wird i. Vak. eingeengt und der Rückstand an Kieselgel chromatographiert, wobei die gewünschte Carbonsäure in einer Ausbeute von 52% erhalten wird.

**19:** 2-[4-(4-Fluor-phenoxy)-benzolsulfonyl]-5-(2-piperidin-1-yl-ethoxy)-1,2,3,4-tetrahydro-isochinolin-1-carbonsäure-hydroxyamid;
Zu einer Lösung von 38 mg (68,5 µmol) 2-[4-(4-Fluoro-phenoxy)-benzolsulfonyl]-5-(2-piperidin-1-yl-ethoxy)-1,2,3,4-tetrahydro-isochinolin-1-carbonsäure und 16,6 µl (151 µmol) N-Metylmorpholin in 2 ml DMF werden bei -20 ˚C 8,16 µl (85,6 µmol) Ethylchloroformat gegeben und 30 Minuten bei -20 ˚C gerührt. Danach wird mit 37,2 µl (274 µmol) O-Trimethylsilyl-hydroxylamin versetzt und die Lösung langsam auf Raumtemperatur erwärmt. Nach Stehen über Nacht wird i. Vak. eingeengt und der Rückstand durch eine Chromatographie an Kieselgel gereinigt, wobei die Titelverbindung als farbloser Feststoff isoliert werden kann. Ausbeute 46%.

**Beispielverbindungen 21 und 22:**

**21:** 8-Brom-2-[4-(4-fluor-phenoxy)-benzolsulfonyl]-5-(2-piperidin-1-yl-ethoxy)-1,2,3,4-tetrahydro-isochinolin-1-carbonsäure-hydroxyamid;

**22:** 8-Brom-2-[4-(4-fluor-phenoxy)-benzolsulfonyl]-5-(2-piperidin-1-yl-ethoxy)-1,2,3,4-tetrahydro-isochinotin-1-carbonsäure;

**21A:** N-[2-(5-Brom-2-methoxy-phenyl)-ethyl]-4-(4-fluor-phenoxy)-benzolsulfonamid; 1,0 g (3,2 mmol) 5-Brom-2-methoxy-phenylethylamin-Hydrochlorid werden zusammen mit 1,25 g (9,6 mmol) Hünigs Base in 10 ml Dichlormethan vorgelegt und bei 0 ˚C mit einer Lösung von 1,02 g (3,5 mmol) 4-(4-Fluor-phenoxy)-benzolsulfonylchlorid in 10 ml Dichlormethan versetzt und vier Stunden bei Raumtemperatur gerührt. Zur Aufarbeitung wird mit ca. 30 ml Dichlormethan verdünnt und mit 2 N HCl, 1 N NaOH sowie mit $H_2O$ gewaschen. Die organische Phase wird mit $MgSO_4$ getrocknet und eingeengt. Das so erhaltene Rohprodukt kann ohne weitere Reiningung weiter umgesetzt werden.

**21B:** 8-Brom-2-[4-(4-fluor-phenoxy)-benzolsulfonyl]-5-methoxy-1,2,3,4-tetrahydroisochinoline-1-carbonsäure;

0,81 g (1,7 mmol) N-[2-(5-Brom-2-methoxy-phenyl)-ethyl]-4-(4-fluor-phenoxy)-benzolsulfonamid (Rohprodukt, 21A) werden in 10 ml Trifluoressigsäure mit 0,32 g (3,4 mmol) Glyoxylsäure-Monohydrat bei Raumtemperatur gerührt. Nach 12 Stunden wird auf Eis gegeben und mit Dichlormethan extrahiert. Nach Trocknen mit $MgSO_4$ und Einengen werden 0,84 g der Titelverbindung erhalten, die ohne weitere Reinigung weiter umgesetzt werden können.

**21C:** 8-Brom-2-[4-(4-fluor-phenoxy)-benzolsulfonyl]-5-hydroxy-1,2,3,4-tetrahydroisochinoline-1-carbonsäure;
0,84 g (1,6 mmol) 8-Brom-2-[4-(4-fluor-phenoxy)-benzolsulfonyl]-5-methoxy-1,2,3,4-tetrahydro-isochinoline-1-carbonsäure (Rohprodukt, 21B) werden in 20 ml Dichlormethan gelöst. Bei 0 ˚C wird eine Lösung von 1,19 g (4,7 mmol) Bortribromid in 10 ml Dichlormethan zugetropft und die Lösung langsam auf Raumtemperatur kommen lassen. Nach zwei Stunden wird zweimal mit 2 N HCl und einmal mit $H_2O$ gewaschen, mit $MgSO_4$ getrocknet und vom Lösungsmittel befreit, wobei 0,61 g der Titelverbindung erhalten werden, die ohne weitere Reinigung weiter umgesetzt werden können.

**21D:** 8-Brom-2-[4-(4-fluor-phenoxy)-benzolsulfonyl]-5-hydroxy-1,2,3,4-tetrahydroisochinoline-1-carbonsäure-ethylester;
0,60 g (1,2 mmol) 8-Brom-2-[4-(4-fluor-phenoxy)-benzolsulfonyl]-5-hydroxy-1,2,3,4-tetrahydro-isochinoline-1-carbonsäure (Rohprodukt, 21C) werden in 10 ml Ethanol mit 0,25 ml konz. $H_2SO_4$ versetzt und zum Rückfluß erhitzt. Nach fünf Stunden werden weitere 0,25 ml konz. $H_2SO_4$ zugegeben und eine weitere Stunde zum Rückfluß erhitzt. Zur Aufarbeitung wird eingeengt, der Rückstand in ges. $Na_2CO_3$-Lösung aufgenommen und zweimal mit Dichlormethan extrahiert. Die organischen Phasen werden mit $MgSO_4$ getrocknet und das Lösungsmittel i. Vak. entfernt. Das so erhaltene Rohprodukt kann ohne Reinigung weiter umgesetzt werden.

**21E:** 8-Brom-2-[4-(4-fluor-phenoxy)-benzolsulfonyl]-5-(2-piperidin-1-yl-ethoxy)-1,2,3,4-tetrahydro-isochinolin-1-carbonsäure-ethylester;
0,515 g $PPh_3$ (polymer gebunden, 1,2 - 1,5 mmol/g) werden in 5 ml Dichlormethan mit 0,13 g Diisopropylazodicarboxylat versetzt und 15 min bei Raumtemperatur geschüttelt. Danach werden 0,17 g (0,31 mmol) 8-Brom-2-[4-(4-fluor-phenoxy)-benzolsulfonyl]-5-hydroxy-1,2,3,4-tetrahydro-isochinoline-1-carbonsäure-ethylester (Rohprodukt, 21 D), 0,031 g (0,31 mmol) Triethylamin und 0,04 g (0,31 mmol) 2-Piperidin-1-yl-ethanol zugegeben und bei Raumtemperatur geschüttelt, bis mittels LCMS-Kontrolle keine weitere Umsatzsteigerung mehr festhestellt werden kann. Zur Aufarbeitung wird filtriert und das Filtrat zweimal mit $H_2O$ gewaschen. Die wässrigen Phasen werden zweimal mit Dichlormethan extrahiert. Die vereinigten organischen Phasen mit $MgSO_4$ getrocknet und einrotiert. Das so erhaltene Rohprodukt wird an einer präp. HPLC gereingt, wobei 0,08 g der Titelverbindung als Trifluoracetat erhalten werden.

**22:** 8-Brom-2-[4-(4-fluor-phenoxy)-benzolsulfonyl]-5-(2-piperidin-1-yl-ethoxy)-1,2,3,4-tetrahydro-isochinolin-1-carbonsäure;
0,25 g (0,38 mmol) 8-Brom-2-[4-(4-fluor-phenoxy)-benzolsulfonyl]-5-(2-piperidin-1-yl-ethoxy)-1,2,3,4-tetrahydro-isochinolin-1-carbonsäure-ethylester werden in 7 ml Methanol mit 2 ml 2 N KOH versetzt und bei Raumtemperatur gerührt. Nach zwei
Stunden werden weitere 2 ml 2 N KOH zugegeben und noch eine Stunde gerührt. Zur Aufarbeitung wird das Methanol abrotiert und der wässrige Rückstand mit 2 N HCl auf einen pH-Wert von 6-7 eingestellt. Absaugen des Niederschlags und Trocknen liefert 0,13 g der gewünschten Carbonsäure.

**21:** 8-Brom-2-[4-(4-fluor-phenoxy)-benzolsulfonyl]-5-(2-piperidin-1-yl-ethoxy)-1,2,3,4-tetrahydro-isochinolin-1-carbonsäure-hydroxyamid;
0,1 g (0,16 mmol) 8-Brom-2-[4-(4-fluor-phenoxy)-benzolsulfonyl]-5-(2-piperidin-1-yl-ethoxy)-1,2,3,4- tetrahydro-isochinolin-1-carbonsäure werden nach der unter Beispiel 19 beschriebenen Methode in die gewünschte Hydroxamsäure 21 überführt, wobei nach Reinigung an einer präp. HPLC die Titelverbindung als Trifluoracetat erhalten wird.

Beispiel 2: Katalytische Inhibition der Gelatinase-B (MMP-9) in humanem Serum durch synthetische MMP-Inhibitoren

Frisch isoliertes humanes Serum wurde in den gewünschten Volumen in flüssigem $N_2$ schockgefroren und anschließend bei -80˚C gelagert. Für die katalytische Inhibition von MMP-9 in humanem Serum wurden die entsprechenden Volumenanteile an humanem Serum bei Raumtemperatur aufgetaut. 100 Volumenanteile unverdünntes, frisch aufgetautes humanes Serum wurde mit 1 Volumenanteil Testsubstanz versetzt, gut durchmischt und 1 h bei 37˚C inkubiert. Die Testsubstanzen wurden in 100% Dimethylsulfoxid gelöst und eine 10 mM Stammlösung hergestellt. Für Dosis-Wirkungskurven wurden die Testsubstanzen in Endkonzentrationen von 200μM bis 0.001 μM auf ihre katalytische Inhibition von MMP-9 in humanem Serum überprüft. Die Endkonzentration von DMSO in der Reaktionslösung war jeweils

1 %.

Aktive MMP-9 aus den oben beschriebenen Reaktionslösungen wurde mit Hilfe eines MMP-9 Aktivität-Assay-Systems (Amersham Pharmacia Biotech Europe GmbH) bestimmt.

Nach der Inkubation wurde ein Volumenanteil des oben beschriebenen Reaktionsgemisches mit 20 Volumenanteilen MMP-9 Reaktionspuffer verdünnt. Die so verdünnten Proben wurden auf eine mit einem spezifischen MMP-9 Antikörper beschichteten 96er Testplatte (Amersham Pharmacia Biotech Europe GmbH, Deutschland) übertragen und über Nacht bei 4˚C inkubiert. Des weiteren wurden verschiedene Konzentrationen an gelöstem, rekombinanten humanem pro MMP-9 zur Erstellung der Standardkurve ebenfalls auf die 96er Testplatte übertragen.

Pro Loch wurden 100μL der jeweiligen Proben eingesetzt. Sämtliches in den Proben vorhandenes MMP-9 (proMMP-9; aktives MMP-9 im Komplex oder nicht im Komplex mit der jeweiligen Testsubstanz), wurde durch den spezifischen MMP-9 Antikörper aus dem Probengemisch isoliert und an eine feste Matrix gebunden.

Die immobilisierten MMP-9 Proben wurden nach der Inkubation 4x mit jeweils 200μL Waschpuffer gewaschen. Anschließend wurde vorhandenes proMMP-9 mit 50μL APMA-Lösung für 1.5 h bei 37˚C aktiviert.

Zur Messung der Enzymaktivität wurden nach der Inkubation zu jeder Probe 50μL Detektionsreagenz hinzugefügt und gemischt. Die Absorption der so beschickten Testplatte wurde sofort bei 405 nm in einem ELISA Messgerät gemessen, um den Zeitpunkt t=0 festzulegen. Anschließend wurde die Testplatte 2 bis 3 h bei 37˚C inkubiert und danach die Absorption bei 405 nm vermessen.

Mittels der eingesetzten Standardkurve wurden die erhaltenen Messwerte wie folgt umgerechnet auf die verbleibende, nicht inhibierte aktive Form von MMP-9 in ng/mL.

$$X = (Y - b) / m * 20$$

X = aktives MMP-9 in [ng/mL]
Y = gemessene Absorption
b = Schnittpunkt y-Achse
m = Steigung
20 = Verdünnunsgfaktor

Die Inhibitorwirkung wurde als prozentuale Hemmung nach folgender Formel berechnet:

$$\% \text{ Hemmung} = 100 - (X_2 * 100 / X_1)$$

$X_1$ = aktives MMP-9 in [ng/mL] ohne Testsubstanz
$X_2$ = aktives MMP-9 in [ng/mL] mit Testsubstanz

Der $IC_{50}$, diejenige Inhibitorkonzentration, welche für eine 50 %ige Hemmung der Enzymaktivität der erforderlich ist, wurde graphisch durch Auftragen der prozentualen Hemmungen bei verschiedenen Inhibitorkonzentrationen ermittelt.

Die Zusammensetzung der jeweiligen eingesetzten Pufferlösungen war wie folgt:

Reaktionspuffer:

50mM Tris-HCl pH 7.6
1.5mM NaCl
0.5mM $CaCl_2$
1μM $ZnCl_2$
0.01% (v/v) BRIJ 35

Eingesetztes, rekombinantes MMP-9 für Standard:

0; 0.5; 1.0; 2.0; 4.0; 8.0; 16.0 ng/ml

Waschpuffer:

0.01 M Natriumphosphat-Puffer pH 7.0
0.05% Tween20

APMA-Lösung:

1 mM p-Aminophenyl-Mercuric-Acetat
50mM Tris-HCl pH 7.6
1.5mM NaCl
0.5mM $CaCl_2$
1$\mu$M $ZnCl_2$
0.01% (v/v) BRIJ 35

Detektionsreagenz:

Modifizierte Urokinase
S-2444 Peptid- Substrat
50mM Tris-HCl pH 7.6
1.5mM NaCl
0.5mM $CaCl_2$
1$\mu$M $ZnCl_2$
0.01% (v/v) BRIJ 35

Beispiel 3: Bestimmung der Löslichkeit mittels CLND (Chemo Lumineszens Nitrogen Detetector)

Zur thermodynamischen Löslichkeitsmessung wird 1 mg Festsubstanz in 250 $\mu$l PBS Puffer (Natrium Phosphat 10 mM; pH 7,4; 0,9 % NaCl isotonische Salzkonzentration) aufgenommen, gemischt und die Suspension wird bei Raumtemperatur 16 Stunden geschüttelt. Anschließend wird die Lösung bei 14 000 rpm 5 Minuten lang zentrifugiert. Die Lösung wird vom Pellet abgenommen und Ober einen Spritzenfilter (0,45 $\mu$m Porengröße) filtriert. Es wird kontrolliert, ob ein Pellet vorhanden ist, um sicher zu gehen, dass eine gesättigte Lösung vorlag. Die filtrierte klare Lösung wird mittels HPLC analysiert.

Die Quantifizierung der gelösten Substanzmenge erfolgt mit einer generischen chromatographischen Methode:

Entweder wird eine Kalibriergerade aus DMSO Stocklösungen (1-500 $\mu$M) der entsprechenden Substanz zur Quantifizierung des entsprechenden Signals im UV (254 nm) Chromatogram der Löslichkeitsprobe verwendet oder die Quantifizierung wird über Peakfläche in der CLND Spur vorgenommen. Im Fall der CLND Quantifizierung wird Coffein als Standard in einer Kalibrierverdünnung verwendet.

Die Quantifizierungsgrenze wird mit der Kalibrierreihe zusammen bestimmt. Üblicherweise liegt die Quantifizierungsgrenze bei 1-5 $\mu$M bei UV Detektion, 5-10 $\mu$M bei CLND Quantifizierung (abhängig von der Anzahl der Stickstoffatome im Molekül bzw. des Extinktionskoeffizienten des Moleküls). Liegt die Signalintensität des gelösten Moleküls unter der Quantifizierungsgrenze wird als Ergebnis kleiner als die Quantifizierungsgrenze angegeben.

Die klare Lösung nach 16 Stunden Inkubation wird immer als Duplicate gemessen, einmal 40-fach verdünnt und einmal direkt ohne Verdünnung. Die Probe, bei der das Signal im linearen Bereich der Kalibrierung liegt wird zur Bestimmung der Löslichkeit herangezogen.

Tabelle 3: Analytische Daten von Beispielverbindungen der Formel (I)

| Beispielverb. | Name | Retentionszeit [min] | Masse, $M+H^+$ [g/mol] | LCMS-Methode |
|---|---|---|---|---|
| 1 | 2-[4-(4-Fluoro-phenoxy)-benzolsulfonyl]-6-(2-piperidin-1-yl-ethoxy)-1,2,3,4-tetrahydro-isoquinoline-1-carbonsäure hydroxyamide | 3,27 | 570,2 | A |
| 2 | 2-[4-(4-Fluoro-phenoxy)-benzolsulfonyl]-6-(2-piperidin-1-yl-ethoxy)-1,2,3,4-tetrahydro-isoquinoline-1-carbonsäure | 1,22 | 555,2 | D |
| 3 | 2-[4-(4-Fluoro-phenoxy)- 2,95 benzolsulfonyl]-6-[3-(4-methyl-piperazin-1-yl)-propoxy]-1,2,3,4- tetrahydro-isoquinoline-1-carbonsäure hydroxyamide | | 599,3 | A |

(fortgesetzt)

| Beispielverb. | Name | Retentionszeit [min] | Masse, M+H$^+$ [g/mol] | LCMS-Methode |
|---|---|---|---|---|
| 4 | 2-[4-(4-Fluoro-phenoxy)-benzolsulfonyl]-6-[3-(4-methyl-piperazin-1-yl)-propoxy]-1,2,3,4- tetrahydro-isoquinoline-1-carbonsäure | 3,94 | 584,5 | B |
| 5 | 2-[4-(4-Fluoro-phenoxy)-benzolsulfonyl]-6-(2-methoxy-ethoxy)-1,2,3,4-tetrahydro-isoquinoline-1-carbonsäure hydroxyamid | 3,76 | 517,2 | A |
| 6 | 2-[4-(4-Fluoro-phenoxy)-benzolsulfonyl]-6-(2-methoxy-ethoxy)-1,2,3,4-tetrahydro-isoquinoline-1-carbonsäure | 4,09 | 502,2 | A |
| 7 | 2-[4-(4-Fluoro-phenoxy)-benzolsulfonyl]-6-(2-morpholin-4-yl-ethoxy)-1,2,3,4-tetrahydro-isoquinoline-1-carbonsäure hydroxyamide | 3,66 | 572,5 | B |
| 8 | 2-[4-(4-Fluoro-phenoxy)-benzolsulfonyl]-6-(2-morpholin-4-yl-ethoxy)-1,2,3,4-tetrahydro-isoquinoline-1-carbonsäure | 3,90 | 557,4 | B |
| 9 | 6-(2-Diethylamino-ethoxy)-2-[4-(4-fluoro-phenoxy)-benzolsulfonyl]-1,2,3,4-tetrahydro-isoquinoline-1-carbonsäure hydroxyamide | 3,23 | 558,1 | A |
| 10 | 6-(2-Diethylamino-ethoxy)-2-[4-(4-fluoro-phenoxy)-benzolsulfonyl]-1,2,3,4-tetrahydro-isoquinoline-1-carbonsäure | 3,89 | 543,1 | A |
| 11 | 2-[4-(4-Fluoro-phenoxy)-benzolsulfonyl]-6-(2-imidazol-1-yl-ethoxy)-1,2,3,4-tetrahydro-isoquinoline-1-carbonsäure hydroxyamide | 3,08 | 553,0 | A |
| 12 | 2-[4-(4-Fluoro-phenoxy)-benzolsulfonyl]-6-(2-imidazol-1-yl-ethoxy)-1,2,3,4-tetrahydro-isoquinoline-1-carbonsäure | 3,30 | 538,0 | A |
| 13 | 2-[4-(4-Methoxy-phenoxy)-benzolsuhonyl]-6-(2-piperidin-1-yl-ethoxy)-1,2,3,4-tetrahydro-isoquinoline-1-carbonsäure hydroxyamide | 3,96 | 582,6 | B |
| 14 | 2-[4-(4-Methoxy-phenoxy)-benzolsulfonyl]-6-(2-piperidin-1-yl-ethoxy)-1,2,3,4-tetrahydro-isoquinoline-1-carbonsäure | 4,15 | 567,5 | B |
| 15 | 6-(2-Diethylamino-ethoxy)-2-[4-(4-methoxy-phenoxy)-benzolsuhonyl]-1,2,3,4-tetrahydro-isoquinoline-1-carbonsäure hydroxyamide | 3,19 | 570,2 | A |
| 16 | 6-(2-Diethylamino-ethoxy)-2-[4-(4-methoxy-phenoxy)-benzolsulfonyl]-1,2,3,4-tetrahydro-isoquinoline-1-carbonsäure | 4,21 | 555,5 | B |

(fortgesetzt)

| Beispielverb. | Name | Retentionszeit [min] | Masse, $M+H^+$ [g/mol] | LCMS-Methode |
|---|---|---|---|---|
| 17 | 6-(2-Imidazol-1-yl-ethoxy)-2-[4-(4-methoxy-phenoxy)-benzolsulfonyl]-1,2,3,4-tetrahydro-isoquinoline-1-carbonsäure hydroxyamide | 3,12 | 565,2 | A |
| 18 | 6-(2-Imidazol-1-yl-ethoxy)-2-[4-(4-methoxy-phenoxy)-benzolsulfonyl]-1,2,3,4-tetrahydro-isoquinoline-1-carbonsäure | 4,13 | 550,4 | B |
| 19 | 2-[4-(4-Fluoro-phenoxy)-benzolsulfonyl]-5-(2-piperidin-1-yl-ethoxy)-1,2,3,4-tetrahydro-isoquinoline-1-carbonsäure hydroxyamide | 3,19 | 570,3 | A |
| 20 | 2-[4-(4-Fluoro-phenoxy)-benzolsulfonyl]-5-(2-piperidin-1-yl-ethoxy)-1, 2, 3,4-tetrahydro-isoquinoline-1-carbonsäure | 4,25 | 555,5 | B |
| 21 | 8-Bromo-2-[4-(4-fluoro-phenoxy)-benzolsulfonyl]-5-(2-piperidin-1-yl-ethoxy)-1,2,3,4- tetrahydro-isoquinoline-1-carbonsäure hydroxyamide | 1,40 | 648,3/650,4 | C |
| 22 | 8-Bromo-2-[4-(4-fluoro-phenoxy)-benzolsulfonyl]-5-(2-piperidin-1-yl-ethoxy)-1,2,3,4- tetrahydro- isoquinoline-1- carbonsäure | 1,53 | 633,3/635,3 | C |

LCMS-Methoden:

| | |
|---|---|
| A | MS-Gerät: LCT/Ionization: ESI+;<br>Stationäre Phase: Hypersil C18;<br>Mobile Phase: (ACN) : ($H_2O$ + 0,05% TFA), 5:95 (0 min) to 95:5 (5 min) to 95:5 (5,5 min) to 95:5 (7 min);<br>Flußrate: 1 ml/min;<br>Temperatur: 30 ˚C |
| B | MS-Gerät: MUX/Ionization: ESI+;<br>Stationäre Phase: Atlantis C18;<br>Mobile Phase: (ACN) : ($H_2O$ + 0,05% TFA),5:95 (0 min) to 95:5 (5 min) to 95:5 (5,5 min) to 95:5 (7 min);<br>Flußrate: 1 ml/min;<br>Temperatur: 30 ˚C |
| C | MS-Gerät: LCT/Ionization: ESI+;<br>Stationäre Phase: Col YMC J'sphere 33x2;<br>Mobile Phase: (ACN + 0,05% TFA) : ($H_2O$ + 0,05% TFA),5:95 (0 min) to 95:5 (2,5 min) to 95:5 (3 min);<br>Flußrate: 1 ml/min;<br>Temperatur: 30 ˚C |
| D | MS-Gerät: LCT/Ionization: ESI+;<br>Stationäre Phase: Col YMC J'sphere ODS H80 20x2;<br>Mobile Phase: (ACN) : ($H_2O$ + 0,05% TFA), 4:96 (0 min) to 95:5 (2,0 min) to 95:5 (2,4 min);<br>Flußrate: 1 ml/min;<br>Temperatur: 30 ˚C |

**Patentansprüche**

1. Verbindung der Formel (I)

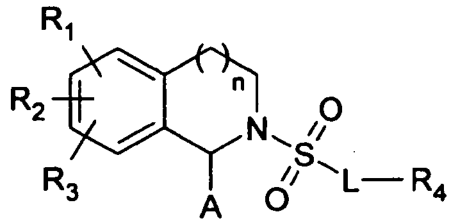

(I)

wobei

$R_1$, $R_2$ und $R_3$ unabhängig voneinander H, F, Cl, Br, CN, OH, $(C_1-C_6)$Alkyl, $(C_3-C_8)$Cycloalkyl, $O(C_1-C_6)$Alkyl, $O(C_3-C_8)$Cycloalkyl, $OC(O)-(C_1-C_6)$Alkyl, $OC(O)-(C_3-C_8)$Cycloalkyl, $C(O)O-(C_1-C_6)$Alkyl, $C(O)O-(C_3-C_8)$Cycloalkyl, $C(O)NR_5R_6$, $NR_5R_6$, $NR_5C(O)R_6$ oder eine Gruppe X-Q-Y sind, wobei

X eine kovalente Bindung, O oder $NR_7$ ist,
Q $(C_1-C_4)$Alkylen ist,
Y $OR_8$, $NR_8R_9$, $C(O)OR_8$, $S(O)_2OR_8$, $S(O)_2NR_8R_9$, ein fünf- oder sechsgliedriger gesättigter Heterozyklus mit 1 oder 2 N- und/oder O-Atomen ist, in dem die N-Atome mit H oder $(C_1-C_6)$-Alkyl substituiert sind, oder Y ein fünf- oder sechsgliedriger aromatischer Heterozyklus mit 1, 2 oder 3 N-Atomen ist,

wobei 1 oder 2, vorzugsweise ein Rest $R_1$, $R_2$ oder $R_3$ durch X-Q-Y beschrieben wird,

A $C(O)OR_{10}$, $C(O)NR_{10}R_{11}$, oder $C(O)NR_{10}OH$ ist,
n 1 ist,
L definiert wird durch eine kovalente Bindung oder $(C_1-C_4)$Alkylen,
$R_4$ Phenyl oder $(C_5-C_{14})$Heteroaryl ist, wobei der Phenyl- oder $(C_5-C_{14})$Heteroarylrest substituiert ist mit einer Gruppe T-Z, wobei

T definiert ist durch eine kovalente Bindung, O, NH oder $N(C_1-C_4)$Alkyl,
Z ausgewählt ist aus der Gruppe Phenyl, $(C_5-C_{14})$Heteroaryl oder $(C_3-C_8)$Heterocydoalkyl, wobei Phenyl, $(C_5-C_{14})$Heteroaryl oder $(C_3-C_8)$Heterocycloalkyl unsubstituiert ist oder substituiert ist mit 1, 2 oder 3 Substituenten unabhängig voneinander ausgewählt aus der Gruppe F, Cl, Br, CN, OH, $(C_1-C_6)$Alkyl, $SO_2(C_1-C_6)$Alkyl, O- $(C_1-C_4)$Alkylen-O-$(C_1-C_6)$Alkyl, O-$(C_1-C_6)$Alkyl, $(C_3-C_8)$Cycloalkyl, $O(C_3-C_8)$Cycloalkyl, $(C_2-C_6)$Alkinyl, $O(C_2-C_6)$Alkinyl, oder $NR_{14}R_{15}$, wobei
$R_{14}$ und $R_{15}$ unabhängig voneinander definiert werden durch H, $(C_1-C_6)$Alkyl, $(C_3-C_8)$Cycloalkyl, $(C_2-C_6)$Alkinyl, $C(O)-(C_1-C_6)$Alkyl, $C(O)-O-(C_1-C_6)$Alkyl, $C(O)-NH-(C_1-C_6)$Alkyl, $C(O)-(C_3-C_8)$Cycloalkyl, $C(O)-O-(C_3-C_8)$Cycloalkyl, $C(O)-NH-(C_3-C_8)$Cycloalkyl, $C(O)-(C_2-C_6)$Alkinyl, $C(O)-O-(C_2-C_6)$Alkinyl oder $C(O)-NH-(C_2-C_6)$Alkinyl sind,

$R_5$, $R_6$, $R_7$, $R_8$, $R_9$, $R_{10}$ und $R_{11}$ unabhängig voneinander H oder $(C_1-C_6)$Alkyl bedeuten, und

wobei optional unabhängig voneinander ein oder mehrere H-Atome in $(C_1-C_6)$Alkyl-, $(C_1-C_6)$Alkylen-, $(C_1-C_4)$Alkyl-, $(C_1-C_4)$Alkylen-, $(C_2-C_6)$Alkenyl-, $(C_3-C_8)$Cycloalkyl-, $(C_3-C_8)$Cycloalkylen- oder $(C_2-C_6)$Alkinyl-Resten durch F-Atome ersetzt sein können,
oder deren pharmakologisch verträgliche Salze.

2. Verbindung der Formel (I) gemäß Anspruch 1, wobei
$R_1$, $R_2$ und $R_3$ unabhängig voneinander H, F, Cl, Br, CN, OH, $(C_1-C_6)$Alkyl, Cyclopropyl, $O(C_1-C_6)$Alkyl, Acetyl, Propionyl, $C(O)O-(C_1-C_4)$Alkyl, $NR_5R_6$ oder eine Gruppe X-Q-Y sind, wobei

X O,
Q $(C_1-C_4)$Alkylen, und

Y O-($C_1$-$C_4$)Alkyl, $NR_8R_9$, COOH, C(O)O($C_1$-$C_4$)Alkyl, $SO_3H$, $S(O)_2O$($C_1$-$C_4$)Alkyl, $SO_2NR_8R_9$, ein fünf- oder sechsgliedriger gesättigter Heterozyklus mit 1 oder 2 N- und/oder O-Atomen ist, in dem die N-Atome mit H oder ($C_1$-$C_4$)-Alkyl substituiert sind, oder Y ein fünf- oder sechsgliedriger aromatischer Heterozyklus mit 1 oder 2 N-Atomen ist,

wobei ein oder zwei Reste $R_1$, $R_2$ oder $R_3$ durch eine Gruppe X-Q-Y beschrieben werden,

A COOH, $C(O)NH_2$ oder C(O)NHOH ist,
n 1,
L eine kovalente Bindung ist,
$R_4$ Phenyl oder ($C_5$-$C_{10}$)Heteroaryl ist, wobei der Phenyl- oder ($C_5$-$C_{10}$)Heteroarylrest substituiert ist mit einer Gruppe T-Z, wobei
T definiert ist durch eine kovalente Bindung oder O,
Z ausgewählt ist aus der Gruppe Phenyl oder ($C_5$-$C_{10}$)Heteroaryl, wobei Phenyl oder ($C_5$-$C_{14}$)Heteroaryl unsubstituiert ist oder substituiert ist mit 1 oder 2 Substituenten unabhängig voneinander ausgewählt aus der Gruppe F, Cl, Br, CN, OH, ($C_1$-$C_6$)Alkyl, O-($C_1$-$C_4$)Alkylen- O-($C_1$-$C_6$)Alkyl, O-($C_1$-$C_6$)Alkyl, ($C_2$-$C_6$)Alkinyl, O ($C_2$-$C_6$)Alkinyl,
$R_5$ und $R_6$ unabhängig voneinander H oder ($C_1$-$C_6$)Alkyl bedeuten,
$R_8$ und $R_9$ unabhängig voneinander H oder ($C_1$-$C_4$)Alkyl bedeuten.

**3.** Verbindung der Formel (I) gemäß einem der Ansprüche 1 oder 2, wobei
$R_1$, $R_2$ und $R_3$ unabhängig voneinander H, F, Cl, Br, CN, OH, ($C_1$-$C_4$)Alkyl, O($C_1$-$C_6$)Alkyl, oder eine Gruppe X-Q-Y sind, wobei

X O,
Q ($C_1$-$C_4$)Alkylen, und
Y O-($C_1$-$C_4$)Alkyl, $NR_8R_9$, COOH, C(O)O($C_1$-$C_4$)Alkyl, $SO_3H$, $S(O)_2O$($C_1$-$C_4$)Alkyl oder ein Heterozyklus ausgewählt aus der Reihe Piperidinyl, Morpholinyl, Pyrrolidinyl, Imidazolyl, Pyrazolyl, Piperazinyl oder N-Methylpiperazinyl ist,

wobei ein oder zwei Reste $R_1$, $R_2$ oder $R_3$ durch eine Gruppe X-Q-Y beschrieben werden,

A COOH, $C(O)NH_2$ oder C(O)NHOH ist,
n 1,
L eine kovalente Bindung ist,
$R_4$ Phenyl ist, wobei der Phenylrest substituiert ist mit einer Gruppe T-Z, wobei

T definiert ist durch eine kovalente Bindung oder O,
Z ausgewählt ist aus der Gruppe Phenyl, wobei Phenyl unsubstituiert ist oder substituiert ist mit 1 oder 2 Substituenten unabhängig voneinander ausgewählt aus der Gruppe F, Cl, Br, CN, OH, ($C_1$-$C_6$)Alkyl, O-($C_1$-$C_4$)Alkylen-O-($C_1$-$C_6$)Alkyl, O-($C_1$-$C_6$)Alkyl, ($C_2$-$C_6$)Alkinyl, O($C_2$-$C_6$)Alkinyl,
$R_8$ und Rg unabhängig voneinander H oder ($C_1$-$C_4$)Alkyl bedeuten.

**4.** Verbindung der Formel (I) gemäß einem der Ansprüche 1 bis 3, wobei
$R_1$, $R_2$ und $R_3$ unabhängig voneinander H, F, Cl, Br, CN, ($C_1$-$C_4$)Alkyl oder eine Gruppe X-Q-Y sind, wobei

X O,
Q ($C_2$-$C_3$)Alkylen, und
Y O-($C_1$-$C_4$)Alkyl, $NR_8R_9$ oder ein Heterozyklus ausgewählt aus der Reihe Piperidinyl, Morpholinyl, Pyrrolidinyl, Imidazolyl, Pyrazolyl, Piperazinyl oder N-Methyl-piperazinyl ist,
wobei ein Rest $R_1$, $R_2$ oder $R_3$ durch eine Gruppe X-Q-Y beschrieben wird,

A COOH oder C(O)NHOH ist,
n 1,
L eine kovalente Bindung ist,
$R_4$ Phenyl ist, wobei der Phenylrest substituiert ist mit einer Gruppe T-Z, wobei

T O ist, und

Z eine Phenylgruppe ist, die unsubstituiert ist oder substituiert ist mit 1 oder 2 Substituenten unabhängig voneinander ausgewählt aus der Gruppe H, F, Cl, CN, OH, $(C_1-C_4)$Alkyl, $O(C_1-C_6)$Alkyl, vorzugsweise F oder$O(C_1-C_6)$Alkyl, und

$R_8$ und $R_9$ unabhängig voneinander H oder $(C_1-C_4)$Alkyl bedeuten.

**5.** Verfahren zur Herstellung einer Verbindung der Formel (I), wobei $R_1$-$R_9$, $R_{14,}$ $R_{15}$, n, L, X, Q, Y, T und Z die in Anspruch 1 angegebene Bedeutung haben, **dadurch gekennzeichnet dass**
in Schritt 1 ein Tetrahydroisochinolin der Formel (XXII)

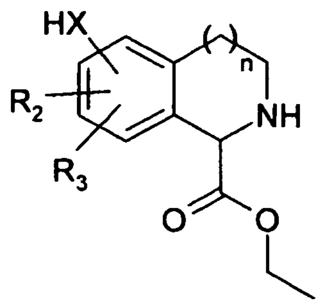

(XXII)

mit einem Sulfonsäurechlorid (III) umgesetzt wird,

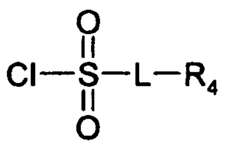

(III)

wobei das Sulfonamid (XXIII) gebildet wird

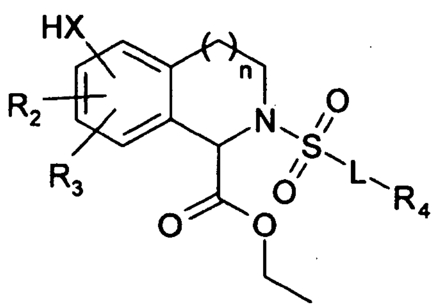

(XXIII),

in Schritt 2 die Verbindung (XXIII) mit einem Dialkylazodicarboxylat und Triphenylphosphin und einer Verbindung HO-Q-Y (V) zur Verbindung (XXIV) umgesetzt wird,

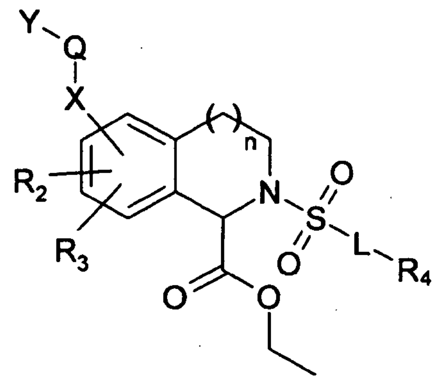

(XXIV),

in Schritt 3 die Estergruppe in der Verbindung (XXIV) mittels einer Base verseift wird, wobei sich eine Verbindung der Formel (I) bildet, in der A $C(O)OR_{10}$ ist und $R_{10}$ gleich H ist,
und optional in Schritt 4 mittels saurer Veresterung durch Umsetzung mit einem $(C_1\text{-}C_6)$Alkylchlorid eine Verbindung der Formel (I) hergestellt wird, in der A gleich $C(O)OR_{10}$ ist und $R_{10}$ gleich $(C_1\text{-}C_6)$Alkyl ist,
oder optional in Schritt 5 durch Umsetzung mit Cl-COOEt und anschließender Zugabe von $R_{10}$HNOTMS eine Verbindung der Formel (I) hergestellt wird, in der A gleich $C(O)NR_{10}OH$ ist,
oder optional in Schritt 6 ein Verbindung der Formel (I) hergestellt wird, in der A gleich $CH_2SH$ ist, wobei die Carbonsäure zunächst mit einem Hydrid reduziert wird und in einem Folgeschritt der dabei intermediär entstandene Alkohol mit einer Abgangsgruppe versehen wird, die durch anschließende Reaktion mit einem $SH^-$ das entsprechenden Thiol liefert,
oder optional in Schritt 7 ein Verbindung der Formel (I) hergestellt wird, in der A gleich $C(O)NR_{10}R_{11}$ ist, wobei die Carbonsäure in Gegenwart einer Base mit einem Amin $(C_1\text{-}C_6)$Alkyl-$NH_2$ umgesetzt wird.

**6.** Verwendung einer Verbindung gemäß einem der Ansprüche 1 bis 4 zur Herstellung eines Arzneimittels.

**7.** Verwendung einer Verbindung gemäß einem der Ansprüche 1 bis 4 zur Herstellung eines Arzneimittels zum Remodelling des Herzens nach einem Herzinfarkt und Atherosklerose, und zur Behandlung von instabiler Angina Pectoris, Herzversagen, Stenose, septischer Schock, Entzündungen, Krebserkrankungen, Tumormetastasenbildung, Kachexie, Anorexie, Ulceration, degenerativen Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen, von Erkrankungen des Bindegewebes wie Kollagenosen, Periodontalerkrankungen, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels, und zur Prophylaxe von Myokard- und Cerebralinfarkten.

**8.** Arzneimittel, **gekennzeichnet durch** einen wirksamen Gehalt an mindestens einer Verbindung der Formel (I) und/oder eines physiologisch verträglichen Salzes gemäß einem der Ansprüche 1 bis 4, zusammen mit einem pharmazeutisch geeigneten und physiologisch verträglichen Trägerstoff, Zusatzstoff und/oder anderen Wirk- und Hilfsstoffen.

**9.** Verfahren zur Herstellung eines Arzneimittels gemäß Anspruch 8, **dadurch gekennzeichnet, dass** man mindestens eine Verbindung der Formel (I) mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

## Claims

**1.** A compound of the formula (I)

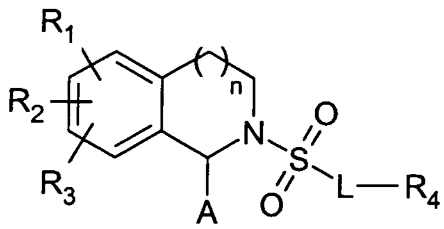

(I)

where

$R_1$, $R_2$ and $R_3$ are independently of one another H, F, Cl, Br, CN, OH, $(C_1-C_6)$alkyl, $(C_3-C_8)$cycloalkyl, $O(C_1-C_6)$alkyl, $O(C_3-C_8)$cycloalkyl, $OC(O)-(C_1-C_6)$alkyl, $OC(O)-(C_3-C_8)$cycloalkyl, $C(O)O-(C_1-C_6)$alkyl, $C(O)O-(C_3-C_8)$cycloalkyl, $C(O)NR_5R_6$, $NR_5R_6$, $NR_5C(O)R_6$ or a group X-Q-Y, where

X is a covalent bond, 0 or $NR_7$,
Q is $(C_1-C_4)$alkylene,
Y is $OR_8$, $NR_8R_9$, $C(O)OR_8$, $S(O)_2OR_8$, $S(O)_2NR_8R_9$, a five- or six-membered saturated heterocycle having 1 or 2 N and/or 0 atoms, in which the N atoms are substituted by H or $(C_1-C_6)$-alkyl, or Y is a five- or six-membered aromatic heterocycle having 1, 2 or 3 N atoms,

where 1 or 2, preferably one radical $R_1$, $R_2$ or $R_3$ is described by X-Q-Y,

A is $C(O)OR_{10}$, $C(O)NR_{10}R_{11}$ or $C(O)NR_{10}OH$,
n is 1,
L is defined by a covalent bond or $(C_1-C_4)$alkylene,
$R_4$ is phenyl or $(C_5-C_{14})$heteroaryl, where the phenyl or $(C_5-C_{14})$heteroaryl radical is substituted by a group T-Z, where
T is defined by a covalent bond, 0, NH or $N(C_1-C_4)$alkyl,
Z is selected from the group of phenyl, $(C_5-C_{14})$heteroaryl or $(C_3-C_8)$heterocycloalkyl, where phenyl, $(C_5-C_{14})$heteroaryl or $(C_3-C_8)$heterocycloalkyl is unsubstituted or is substituted by 1, 2 or 3 substituents independently of one another selected from the group of F, Cl, Br, CN, OH, $(C_1-C_6)$alkyl, $SO_2(C_1-C_6)$alkyl, $O-(C_1-C_4)$alkylene-$O-(C_1-C_6)$alkyl, 0- $(C_1-C_6)$alkyl, $(C_3-C_8)$cycloalkyl, $O(C_3-C_8)$cycloalkyl, $(C_2-C_6)$alkynyl, $O(C_2-C_6)$alkynyl, or $NR_{14}R_{15}$, where
$R_{14}$ and $R_{15}$ are defined independently of one another by H, $(C_1-C_6)$alkyl, $(C_3-C_8)$cycloalkyl, $(C_2-C_6)$alkynyl, $C(O)-(C_1-C_6)$alkyl, $C(O)-O-(C_1-C_6)$alkyl, $C(O)-NH-(C_1-C_6)$alkyl, $C(O)-(C_3-C_8)$cycloalkyl, $C(O)-O-(C_3-C_8)$cycloalkyl, $C(O)-NH-(C_3-C_8)$cycloalkyl, $C(O)-(C_2-C_6)$alkynyl, $C(O)-O-(C_2-C_6)$alkynyl or $C(O)-NH-(C_2-C_6)$alkynyl,

$R_5$, $R_6$, $R_7$, $R_8$, $R_9$, $R_{10}$ and $R_{11}$ are independently of one another H or $(C_1-C_6)$alkyl, and

where optionally independently of one another one or more H atoms in $(C_1-C_6)$alkyl, $(C_1-C_6)$alkylene, $(C_1-C_4)$alkyl, $(C_1-C_4)$alkylene, $(C_2-C_6)$alkenyl, $(C_3-C_8)$cycloalkyl, $(C_3-C_8)$cycloalkylene or $(C_2-C_6)$alkynyl radicals may be replaced by F atoms,
or the pharmacologically acceptable salts thereof.

**2.** The compound of the formula (I) as claimed in claim 1, where
$R_1$, $R_2$ and $R_3$ are independently of one another H, F, Cl, Br, CN, OH, $(C_1-C_6)$alkyl, cyclopropyl, $O(C_1-C_6)$alkyl, acetyl, propionyl, $C(O)O-(C_1-C_4)$alkyl, $NR_5R_6$ or a group X-Q-Y, where

X is O,
Q is $(C_1-C_4)$alkylene, and
Y is $O-(C_1-C_4)$alkyl, $NR_8R_9$, COOH, $C(O)O(C_1-C_4)$alkyl, $SO_3H$, $S(O)_2O(C_1-C_4)$alkyl, $SO_2NR_8R_9$, a five- or six-membered saturated heterocycle having 1 or 2 N and/or O atoms, in which the N atoms are substituted by H or $(C_1-C_4)$-alkyl, or Y is a five- or six-membered aromatic heterocycle having 1 or 2 N atoms,
where one or two radicals $R_1$, $R_2$ or $R_3$ are described by
a group X-Q-Y,

A is COOH, $C(O)NH_2$ or C(O)NHOH,
n is 1,
L is a covalent bond,
$R_4$ is phenyl or ($C_5$-$C_{10}$)heteroaryl, where the phenyl or ($C_5$-$C_{10}$)heteroaryl radical is substituted by a group T-Z, where

T is defined by a covalent bond or O,
Z is selected from the group of phenyl or ($C_5$-$C_{10}$)heteroaryl, where phenyl or ($C_5$- $C_{14}$)heteroaryl is unsubstituted or is substituted by 1 or 2 substituents independently of one another selected from the group of F, Cl, Br, CN, OH, ($C_1$-$C_6$)alkyl, O-($C_1$-$C_4$)alkylene-O-($C_1$-$C_6$)alkyl, O-($C_1$-$C_6$)alkyl, ($C_2$-$C_6$)alkynyl, O ($C_2$-$C_6$)alkynyl,

$R_5$ and $R_6$ are independently of one another H or ($C_1$-$C_6$)alkyl,
$R_8$ and $R_9$ are independently of one another H or ($C_1$-$C_4$)alkyl.

**3.** The compound of the formula (I) as claimed in either of claims 1 or 2, where
$R_1$, $R_2$ and $R_3$ are independently of one another H, F, Cl, Br, CN, OH, ($C_1$-$C_4$)alkyl, O($C_1$-$C_6$)alkyl, or a group X-Q-Y, where

X is O,
Q is ($C_1$-$C_4$)alkylene, and
Y is O-($C_1$-$C_4$)alkyl, $NR_8R_9$, COOH, C(O)O($C_1$- $C_4$)alkyl, $SO_3H$, $S(O)_2O$($C_1$-$C_4$)alkyl or a heterocycle selected from the series piperidinyl, morpholinyl, pyrrolidinyl, imidazolyl, pyrazolyl, piperazinyl or N- methylpiperazinyl, where one or two radicals $R_1$, $R_2$ or $R_3$ are described by a group X-Q-Y,

A is COOH, $C(O)NH_2$ or C(O)NHOH,
n is 1,
L is a covalent bond,
$R_4$ is phenyl, where the phenyl radical is substituted by a group T-Z, where

T is defined by a covalent bond or O,
Z is selected from the group of phenyl, where phenyl is unsubstituted or is substituted by 1 or 2 substituents independently of one another selected from the group of F, Cl, Br, CN, OH, ($C_1$-$C_6$)akyl, O-($C_1$-$C_4$)alkylene-O-($C_1$-$C_6$)alkyl, O- ($C_1$-$C_6$)alkyl, ($C_2$-$C_6$)alkynyl, O($C_2$-$C_6$)alkynyl,

$R_8$ and $R_9$ are independently of one another H or ($C_1$-$C_4$)alkyl.

**4.** The compound of the formula (I) as claimed in any of claims 1 to 3, where
$R_1$, $R_2$ and $R_3$ are independently of one another H, F, Cl, Br, CN, ($C_1$-$C_4$)alkyl or a group X-Q-Y, where

X is O,
Q is ($C_2$-$C_3$)alkylene, and
Y is O-($C_1$-$C_4$)alkyl, $NR_8R_9$ or a heterocycle selected from the series piperidinyl, morpholinyl, pyrrolidinyl, imidazolyl, pyrazolyl, piperazinyl or N-methylpiperazinyl,
where a radical $R_1$, $R_2$ or $R_3$ is described by a group X-Q-Y,
A is COOH or C(O)NHOH,
n is 1,
L is a covalent bond,
$R_4$ is phenyl, where the phenyl radical is substituted by a group T-Z, where

T is O, and
Z is a phenyl group, which is unsubstituted or is substituted by 1 or 2 substituents independently of one another selected from the group of H, F, Cl, CN, OH, ($C_1$-$C_4$)alkyl, O($C_1$-$C_6$)alkyl, preferably F or O($C_1$-$C_6$) alkyl, and
$R_8$ and $R_9$ are independently of one another H or ($C_1$- $C_4$)alkyl.

**5.** A process for preparing a compound of the formula (I), where $R_1$-$R_9$, $R_{14}$, $R_{15}$, n, L, X, Q, Y, T and Z have the meaning indicated in claim 1, which comprises

in step 1 reacting a tetrahydroisoquinoline of the formula (XXII)

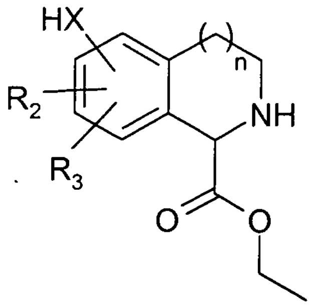

(XXII)

with a sulfonyl chloride (III)

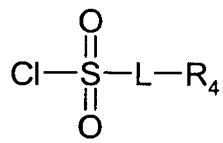

(III)

to form the sulfonamide (XXIII)

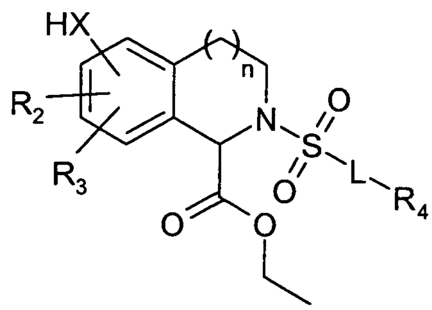

(XXIII),

in step 2 reacting the compound (XXIII) with a dialkyl azodicarboxylate and triphenylphosphine and a compound HO-Q-Y (V) to give the compound (XXIV)

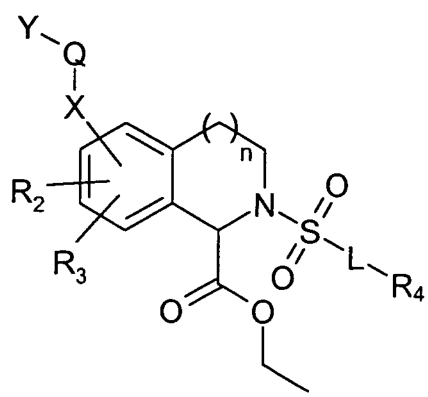

(XXIV),

in step 3 hydrolyzing the ester group in the compound (XXIV) using a base, to form a compound of the formula (I) in which A is $C(O)OR_{10}$, and $R_{10}$ is equal to H,
and optionally in step 4 preparing, by acidic esterification through reaction with a $(C_1-C_6)$alkyl chloride, a compound of the formula (I) in which A is equal to $C(O)OR_{10}$, and $R_{10}$ is equal to $(C_1-C_6)$alkyl,
or optionally in step 5 preparing, by reacting with Cl-COOEt and subsequent addition of $R_{10}$HNOTMS, a compound of the formula (I) in which A is equal to $C(O)NR_{10}OH$,
or optionally in step 6 preparing a compound of the formula (I) in which A is equal to $CH_2SH$, in which case the carboxylic acid is first reduced with a hydride and, in a following step, the alcohol intermediate produced thereby is provided with a leaving group which affords, by subsequent reaction with an $SH^-$, the corresponding thiol,
or optionally in step 7 preparing a compound of the formula (I) in which A is equal to $C(O)NR_{10}R_{11}$, in which case the carboxylic acid is reacted in the presence of a base with an amine $(C_1-C_6)$alkyl-$NH_2$.

**6.** The use of a compound as claimed in any of claims 1 to 4 for producing a medicament.

**7.** The use of a compound as claimed in any of claims 1 to 4 for producing a medicament for remodeling of the heart after a myocardial infarction and atherosclerosis, and for the treatment of unstable angina pectoris, heart failure, stenosis, septic shock, inflammations, cancers, tumor metastasis, cachexia, anorexia, ulceration, degenerative joint disorders such as osteoarthroses, spondyloses, chondrolysis following joint trauma or prolonged joint immobilization after meniscus or patella injuries or ligament tears, of connective tissue disorders such as collagenoses, periodontal disorders, wound-healing disturbances and chronic disorders of the locomotor system such as inflammatory, immunologically or metabolically related acute and chronic arthritides, arthropathies, myalgias and disturbances of bone metabolism, and for the prophylaxis of myocardial and cerebral infarctions.

**8.** A medicament which has an effective content of at least one compound of the formula (I) and/or of a physiologically tolerated salt as claimed in any of claims 1 to 4, together with a pharmaceutically suitable and physiologically tolerated carrier, additive and/or other active ingredients and excipients.

**9.** A process for producing a medicament as claimed in claim 8, which comprises converting at least one compound of the formula (I) with a pharmaceutically suitable and physiologically tolerated carrier and, where appropriate, further suitable active ingredients, additives and excipients into a suitable dosage form.

## Revendications

**1.** Composé de formule (I)

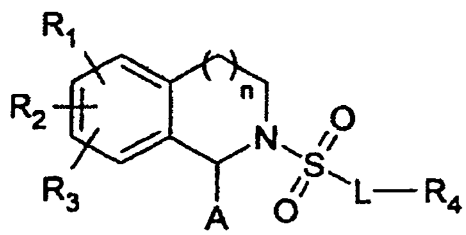

(I)

dans laquelle
$R_1$, $R_2$ et $R_3$ représentent chacun indépendamment des autres H, F, Cl, Br, CN, OH, un radical alkyle en $C_1$-$C_6$, cycloalkyle en $C_3$-$C_8$, O-(alkyle en $C_1$-$C_6$), O-(cycloalkyle en $C_3$-$C_8$), OC(O)-(alkyle en $C_1$-$C_6$) , OC(O)-(cycloalkyle en $C_3$-$C_8$), C(O)O-(alkyle en $C_1$-$C_6$), C(O)O-(cycloalkyle en $C_3$-$C_8$), $C(O)NR_5R_6$, $NR_5R_6$, $NR_5C(O)R_6$, ou un groupe X-Q-Y, où
X est une liaison covalente, O ou $NR_7$,
Q est un radical alkylène en $C_1$-$C_4$,
Y est $OR_8$, $NR_8R_9$, $C(O)OR_8$, $S(O)_2OR_8$ , $S(O)_2NR_8R_9$, un radical hétérocyclique saturé à cinq ou six chaînons, ayant 1 ou 2 atomes N et/ou O, dans lequel les atomes N sont substitués par H ou par un ou des substituants alkyle en $C_1$-$C_6$, ou Y représente un radical hétérocyclique aromatique à cinq ou six chaînons ayant 1, 2 ou 3 atomes N, où 1 ou 2, de préférence un radical $R_1$, $R_2$ ou $R_3$ sont représentés par X-Q-Y,
A est $C(O)OR_{10}$, $C(O)NR_{10}R_{11}$ ou $C(O)NR_{10}OH$,

n vaut 1,
L est défini par une liaison covalente ou un radical alkylène en $C_1$-$C_4$,
$R_4$ est le radical phényle ou un radical hétéroaryle en $C_5$-$C_{14}$, le radical phényle ou hétéroaryle en $C_5$-$C_{14}$ étant substitué par un groupe T-Z, où
T est défini par une liaison covalente, O, NH ou un radical N(alkyle en $C_1$-$C_4$),
Z est choisi dans le groupe comprenant les radicaux phényle, hétéroaryle en $C_5$-$C_{14}$ ou hétérocycloalkyle en $C_3$-$C_8$, les radicaux phényle, hétéroaryle en $C_5$-$C_{14}$ ou hétérocycloalkyle en $C_3$-$C_8$ étant non substitués, ou étant substitués par 1, 2 ou 3 substituants choisis indépendamment les uns des autres dans le groupe consistant en F, Cl, Br, CN, OH, les radicaux alkyle en $C_1$-$C_6$, $SO_2$(alkyle en $C_1$-$C_6$) O-(alkylène en $C_1$-$C_4$)-O-(alkyle en $C_1$-$C_6$), O-(alkyle en $C_1$-$C_6$), cycloalkyle en $C_3$-$C_8$, O-(cycloalkyle en $C_3$-$C_8$), alcynyle en $C_2$-$C_6$, O-(alcynyle en $C_2$-$C_6$) ou $NR_{14}R_{15}$, où $R_{14}$ et $R_{15}$ sont définis, chacun indépendamment de l'autre, par H, un radical alkyle en $C_1$-$C_6$, cycloalkyle en $C_3$-$C_8$, alcynyle en $C_2$-$C_6$, C(O)-(alkyle en $C_1$-$C_6$), C(O)-O-(alkyle en $C_1$-$C_6$), C(O)-NH-(alkyle en $C_1$-$C_6$), C(O)-(cycloalkyle en $C_3$-$C_8$), C(O)-O-(cycloalkyle en $C_3$-$C_8$), C(O)-NH-(cycloalkyle en $C_3$-$C_8$), C(O)-(alcynyle en $C_2$-$C_6$), C(O)-O-(alcynyle en $C_2$-$C_6$) ou C(O)-NH-(alcynyle en $C_2$-$C_6$),
Rs, $R_6$, $R_7$, $R_8$, $R_9$, $R_{10}$ et $R_{11}$ représentent chacun indépendamment des autres H ou un radical alkyle en $C_1$-$C_6$, où, en option, et indépendamment les uns des autres, un ou plusieurs atomes H se trouvant dans les radicaux alkyle en $C_1$-$C_6$, alkylène en $C_1$-$C_6$, alkyle en $C_1$-$C_4$, alkylène en $C_1$-$C_4$, alcényle en $C_2$-$C_6$, cycloalkyle en $C_3$-$C_8$, cycloalkylène en $C_3$-$C_8$ ou alcynyle en $C_2$-$C_6$, peuvent être remplacés par des atomes F,
ou ses sels pharmacologiquement compatibles.

**2.** Composé de formule (I) selon la revendication 1, dans lequel
$R_1$, $R_2$ et $R_3$ représentent chacun indépendamment des autres H, F, Cl, Br, CN, OH, un radical alkyle en $C_1$-$C_6$, cyclopropyle, O(alkyle en $C_1$-$C_6$), acétyle, propionyle, C(O)O-(alkyle en $C_1$-$C_4$), $NR_5R_6$, ou un groupe X-Q-Y, où
X est 0
Q est un radical alkylène en $C_1$-$C_4$,
Y est un radical O-(alkyle en $C_1$-$C_4$), $NR_8R_9$, COOH, C(O)O(alkyle en $C_1$-$C_4$), $SO_3H$, $S(O)_2O$(alkyle en $C_1$-$C_4$), $SO_2NR_8R_9$, un radical hétérocyclique saturé à cinq ou six chaînons et ayant 1 ou 2 atomes N et/ou O, les atomes N étant substitués par H ou un ou des substituants alkyle en $C_1$-$C_4$, ou Y est un radical hétérocyclique aromatique à cinq ou six chaînons ayant 1 ou 2 atomes N, un ou deux radicaux $R_1$, $R_2$ ou $R_3$ étant décrits par un groupe X-Q-Y,
A est COOH, $C(O)NH_2$ ou C(O)NHOH,
n vaut 1,
L est une liaison covalente,
$R_4$ est le radical phényle ou un radical hétéroaryle en $C_5$-$C_{10}$, le radical phényle ou hétéroaryle en $C_5$-$C_{10}$ étant substitué par un groupe T-Z, où
T est défini par une liaison covalente ou O,
Z est choisi dans le groupe consistant en le groupe phényle ou hétéroaryle en $C_5$-$C_{10}$, où le radical phényle ou hétéroaryle en $C_5$-$C_{14}$ est non substitué ou est substitué par un ou deux substituants choisis indépendamment l'un de l'autre dans le groupe consistant en F, Cl, Br, CN, OH, les radicaux alkyle en $C_1$-$C_6$, O-(alkylène en $C_1$-$C_4$)-O-(alkyle en $C_1$-$C_6$), O-(alkyle en $C_1$-$C_6$), alcynyle en $C_2$-$C_6$, O-(alcynyle en $C_2$-$C_6$),
$R_5$ et $R_6$ représentent chacun indépendamment de l'autre H ou un radical alkyle en $C_1$-$C_6$,
$R_8$ et $R_9$ représentent chacun indépendamment de l'autre H ou un radical alkyle en $C_1$-$C_4$.

**3.** Composé de formule (I) selon l'une des revendications 1 ou 2, dans lequel
$R_1$, $R_2$ et $R_3$ représentent chacun indépendamment de l'autre H, F, Cl, Br, CN, OH, un radical alkyle en $C_1$-$C_4$, O-(alkyle en $C_1$-$C_6$) ou un groupe X-Q-Y, où
X est 0,
Q est un radical alkylène en $C_1$-$C_4$, et
Y est un radical O-(alkyle en $C_1$-$C_4$), $NR_8R_9$, COOH, C(O)O(alkyle en $C_1$-$C_4$), $SO_3H$, $S(O)_2O$-(alkyle en $C_1$-$C_4$) ou un radical hétérocyclique choisi dans la série des groupes pipéridinyle, morpholinyle, pyrrolidinyle, imidazolyle, pyrazolyle, pipérazinyle ou N-méthylpipérazinyle,
où un ou deux radicaux $R_1$, $R_2$ ou $R_3$ sont décrits par un groupe X-Q-Y,
A est COOH, $C(O)NH_2$ ou C(O)NHOH,
n vaut 1,
L est une liaison covalente,
$R_4$ est le radical phényle, le radical phényle étant substitué par un groupe T-Z, où
T est défini par une liaison covalente ou O,
Z est choisi dans le groupe comprenant le radical phényle, le radical phényle étant non substitué ou substitué par un ou deux substituants choisis indépendamment l'un de l'autre dans le groupe consistant en F, Cl, Br, CN, OH,

les radicaux alkyle en $C_1$-$C_6$, O-(alkylène en $C_1$-$C_4$)-O-(alkyle en $C_1$-$C_6$), O-(alkyle en $C_1$-$C_6$), alcynyle en $C_2$-$C_6$, O-(alcynyle en $C_2$-$C_6$),
$R_8$ et $R_9$ représentent chacun indépendamment de l'autre H ou un radical alkyle en $C_1$-$C_4$.

**4.** Composé de formule (I) selon l'une des revendications 1 à 3, dans lequel
$R_1$, $R_2$ et $R_3$ représentent chacun indépendamment de l'autre H, F, Cl, Br, CN, un radical alkyle en $C_1$-$C_4$ ou un groupe X-Q-Y, où
X est O,
Q est un radical alkylène en $C_2$-$C_3$, et
Y est un radical O-(alkyle en $C_1$-$C_4$), $NR_8R_9$ ou un radical hétérocyclique choisi dans la série consistant en les groupes pipéridinyle, morpholinyle, pyrrolidinyle, imidazolyle, pyrazolyle, pipérazinyle ou N-méthylpipérazinyle,
où un radical $R_1$, $R_2$ ou $R_3$ est décrit par un groupe X-Q-Y,
A est COOH ou C(O)NHOH,
n vaut 1,
L est une liaison covalente,
$R_4$ est le radical phényle, le radical phényle étant substitué par un groupe T-Z, où
T est O, et
Z est un groupe phényle, qui est non substitué ou est substitué par un ou deux substituants choisis chacun indépendamment de l'autre dans le groupe consistant en H, F, Cl, CN, OH, les radicaux alkyle en $C_1$-$C_4$, O-(alkyle en $C_1$-$C_6$), de préférence F ou les radicaux O-(alkyle en $C_1$-$C_6$), et
$R_8$ et $R_9$ représentent chacun indépendamment de l'autre H ou un radical alkyle en $C_1$-$C_4$.

**5.** Procédé de préparation d'un composé de formule (I), où $R_1$-$R_9$, $R_{14}$, $R_{15}$, n, L, X, Q, Y, T et Z ont les significations données dans la revendication 1, **caractérisé en ce que**
dans l'étape 1, on fait réagir une tétrahydroisoquinoléine de formule (XXII)

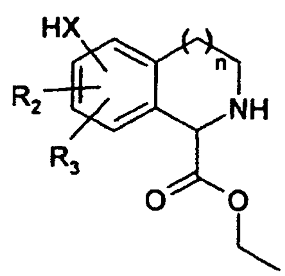

(XXII)

avec un chlorure de sulfonyle (III)

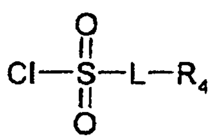

(III)

ce qui provoque la formation du sulfonamide (XXIII)

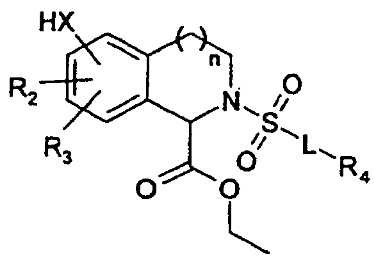

(XXIII),

dans l'étape 2, on fait réagir le composé (XXIII) avec un azodicarboxylate de dialkyle et de la triphénylphosphine et un composé HO-Q-Y (V) pour obtenir le composé (XXIV)

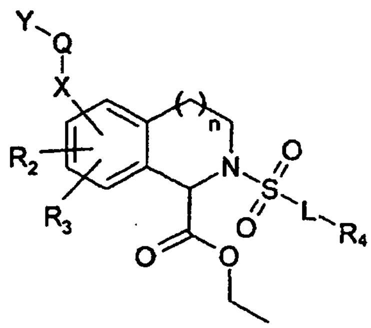

(XXIV),

dans l'étape 3, on saponifie le groupe ester du composé (XXIV) avec une base, avec formation d'un composé de formule (I) dans laquelle A est $C(O)OR_{10}$, et $R_{10}$ est H,
et en option, dans l'étape 4, on prépare par une estérification acide, par réaction avec un chlorure d'alkyle en $C_1$-$C_6$, un composé de formule (I) dans laquelle A est $C(O)OR_{10}$ et $R_{10}$ est un radical alkyle en $C_1$-$C_6$,
ou en option, dans l'étape 5, on prépare par réaction avec du Cl-COOEt puis addition de $R_{10}$HNOTMS, un composé de formule (I) dans laquelle A est $C(O)NR_{10}OH$,
ou en option, dans l'étape 6, on prépare un composé de formule (I) dans laquelle A est $CH_2SH$, l'acide carboxylique étant d'abord réduit avec un hydrure, et, dans une étape subséquente, l'alcool intermédiaire qui se forme à cette occasion est pourvu d'un groupe partant, qui par réaction ultérieure avec un $SH^-$ va donner le thiol correspondant,
ou en option, dans l'étape 7, on prépare un composé de formule (I) dans laquelle A est $C(O)NR_{10}R_{11}$, l'acide carboxylique étant mis à réagir en présence d'une base avec une amine (alkyle en $C_1$-$C_6$)-$NH_2$.

**6.** Utilisation d'un composé selon l'une des revendications 1 à 4 pour la préparation d'un médicament.

**7.** Utilisation d'un composé selon l'une des revendications 1 à 4 pour fabriquer un médicament pour le remodelage du coeur après un infarctus du myocarde et une athérosclérose, et pour le traitement de l'angine de poitrine instable, de l'insuffisance cardiaque, de la sténose, du choc septique, des inflammations, des maladies cancéreuses, de la formation de métastases tumorales, de la cachexie, de l'anorexie, des ulcérations, des maladies dégénératives des articulations telles que les ostéoarthroses, les spondyloses, l'atrophie cartilagineuse après un traumatisme des articulations ou une immobilisation prolongée des articulations après des lésions du ménisque ou de la rotule ou une rupture des ligaments, les maladies du tissu conjonctif telles que les collagénoses, les parodontopathies, les troubles de la cicatrisation et les maladies chroniques de l'appareil moteur, telles que les arthrites aiguës ou chroniques, inflammatoires ou d'origine immunologique ou métabolique, les arthropathies, les myalgies et les troubles du métabolisme osseux, et pour la prophylaxie des infarctus du myocarde et cérébral.

**8.** Médicament, **caractérisé en ce qu'**il a une teneur efficace en au moins un composé de formule (I) et/ou un sel physiologiquement compatible selon l'une des revendications 1 à 4, en même temps qu'un support, un additif et/ou d'autres principes actifs et adjuvants pharmaceutiquement appropriés et physiologiquement compatibles.

**9.** Procédé de fabrication d'un médicament selon la revendication 8, **caractérisé en ce qu'**on met sous une forme posologique appropriée au moins un composé de formule (I) avec un support pharmaceutiquement approprié et physiologiquement compatible, et éventuellement d'autres principes actifs, additifs ou adjuvants appropriés.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9718194 A
- CN 1380288 A
- WO 03016248 A
- DE 102004031850
- US 6153757 A


### In der Beschreibung aufgeführte Nicht-Patentliteratur


- **D. Yip et al.** *Investigational New Drugs,* 1999, vol. 17, 387-399
- **Michaelides et al.** *Current Pharmaceutical Design,* 1999, vol. 5, 787-819
- **S. J. George.** *Exp. Opin. Invest. Drugs,* 2000, vol. 9 (5), 993-1007
- **E. J. M. Creemers et al.** *Circulation Res.,* 2001, vol. 89, 201-210
- *Drugs,* 2001, vol. 61, 1239-1252
- *Bioorg. Med. Chem. Lett.,* 2004, vol. 14, 47-50
- *Current Medicinal Chemistry,* 2001, vol. 8, 425-474
- Remington's Pharmaceutical Sciences. 1985, 1418